# EUROPEAN PATENT SPECIFICATION

(11) **EP 4 346 602 B1**
(45) Date of publication and mention of the grant of the patent: **11.03.2026**
(21) Application number: 22730011.8
(22) Date of filing: 24.05.2022
(51) Int. Cl.: A61B 5/346, A61B 5/00, G16H 50/20

(54) **DYNAMIC AND MODULAR CARDIAC EVENT DETECTION**
DYNAMISCHE UND MODULARE HERZEREIGNISDETEKTION
DÉTECTION DYNAMIQUE ET MODULAIRE D'ÉVÉNEMENTS CARDIAQUES

(30) Priority: 28.05.2021 US 202163194451 P
(43) Date of publication of application: 10.04.2024
(73) Proprietor: Medtronic, Inc., Minneapolis, Minnesota 55432 (US)
(72) Inventor: CHENG, Ya_Jian, Minneapolis, Minnesota 55432 (US)
(74) Representative: Zimmermann & Partner Patentanwälte mbB
(86) International application number: PCT/US2022/030627
(87) International publication number: WO 2022/251145

(56) References cited:
- US-A1- 2020 205 745
- US-A1- 2020 345 261
- US-A1- 2020 352 521
- US-A1- 2020 357 517

## Description

### FIELD

The disclosure relates generally to medical systems and, more particularly, medical systems configured to detect cardiac events.

### BACKGROUND

Some types of medical systems may monitor various data (e.g., cardiac electrophysiological activities and signals) of a patient or a group of patients to detect changes in health. The patient's heart's electrical activity can be detected on the patient's skin or subcutaneously (via an electrocardiogram (ECG)) and inside the heart itself (via a cardiac electrogram (EGM))). In some examples, the medical system may monitor EGM data and/or ECG data to detect one or more types of arrhythmia, such as bradycardia, tachycardia, fibrillation, or asystole (e.g., caused by sinus pause or AV block). In some examples, the medical system may include one or more of an implantable device or a wearable device to collect various measurements used to detect changes in patient health. US 2020/03575517 A1 relates to machine learning based depolarization identification and arrhythmia localization visualization. US2020/0352521 A1 relates to category-based review and reproting of episode data. US2020/0345261 A1 relates to systems, devices, components and methods for detecting the locations of sources of cardiac rhythm disorders in a patient's heart. US2020/0205745 A1 relates to mehtods and systems to configure and use neural networks i ncharacterizing physiological systems.

### SUMMARY

Medical systems and techniques as described herein detect changes in a patient's health (e.g., heart health) based upon data describing that patient's physiology including the patient's cardiac physiology. A medical system including any one or more of a variety of medical devices (e.g., implantable devices, wearable devices, etc.) detects (e.g., evidence of) a cardiac event of a certain type (e.g., an arrhythmia) based on a classification of patient data (e.g., cardiac activity data such as a cardiac EGM or ECG) in accordance with a modular machine learning architecture. As described herein, the medical system performs the classification operation as part of the machine learning algorithm and, based on that operation, determines an appropriate classification (e.g., type) of cardiac event for (e.g., a sample of) the cardiac activity data.

An example medical device, for example, may be implanted into the patient's body in order to monitor the patient heart's electrical activity during which the implanted device receives, as input, cardiac EGM signals representing such electrical activity. The medical device may detect cardiac events based on the cardiac EGM signals and, for each event, store episode data including one or more segments of the cardiac EGM signals. Processing circuitry of the medical system may receive the episode data, and determine whether the cardiac EGM signals provide evidence of an occurrence of a cardiac event episode (e.g., asystole episode) using the classification operations and machine learning algorithms described herein.

Modules of the modular machine learning architecture represent independent functions for the classification operation; in particular, the modular machine learning architecture may configure the modules to classify respective cardiac event types such that each module is dedicated to classifying a different cardiac event type. In addition, the modular machine learning architecture defines each module as an ensemble of component models and, in each module, configures each component model to classify a specific cardiac event type but (e.g., via an application of a integration operation) only outputs, and then stores, one component model's prediction value. The integration operation may mask other component models in that module, causing their resultant prediction values to be discarded. By restricting the output from the modules (and thus, input into a second integration operation for entire architecture) to prediction values corresponding to a component model in each module matching that module's respective cardiac event type, the modular machine learning architecture configures each ensemble as an independent system in the modular machine learning architecture.

The present disclosure includes a number of benefits and advantages that example medical systems and techniques described herein realize by implementing the modular machine learning architecture. Medical systems including medical devices benefit from increased accuracy with respect to detecting episodes of cardiac events that occurred in a patient. As an advantage, such medical systems may improve upon the ability of clinicians to diagnose and treat a patient, and thus on the patient's general health, as well as specifically preventing cardiac events. When the classification operations and machine learning algorithms described herein are implemented on a separate computing system, medical devices may also realize such improvements in event detection without increased resource utilization, especially in consumption of compute resources (e.g., processing cycles) and/or storage resources (e.g., storage space), overall cost, and power consumption.

Medical devices may further benefit from a remote monitoring service (e.g., a cloud computing service). A computing system (e.g., computer servers) may run the remote monitoring service over a network (e.g., Internet) and, via the network, provide application and/or data services to implanted medical devices of patients. When a more recent version of a specific cardiac event type's component model the becomes available (e.g., after government validation), the remote monitoring service may update a corresponding module in the architecture where that component model's prediction is not masked. Other modules do not need to be updated for those modules do not rely on the updated component model. When given patient episode data to classify, the remote monitoring service applies the updated module of the modular machine learning architecture to determine whether to classify the patient episode data as the specific cardiac event type and (if needed) applies the other modules to determine whether to classify the patient episode data as other cardiac event type, according to one example. In another example, the remote monitoring service may communicate the updated module to the medical device for that device to classify the patient episode data; because of the modular nature of the machine learning algorithm and the architecture, the remote monitoring service is not required to update and/or send to the medical device the entire modular machine learning architecture. The independence of each module in the modular machine learning architecture enables the remote monitoring service to send an update consisting only of a minimum portion of a new machine learning model (e.g., a hidden/intermediate neural network layer or a neural network ensemble branch).

In one example, a medical system comprises one or more sensors configured to sense at least one physiological parameter corresponding to cardiac physiology of a patient; sensing circuitry configured to generate patient physiological data based on the sensed physiological parameter, the patient physiological data comprising cardiac EGM data for the patient; and processing circuitry configured to: detect a cardiac event type for the patient based on a classification of the patient physiological data in accordance with a modular machine learning architecture, wherein the modular machine learning architecture comprises, for each of a plurality of cardiac event types, an ensemble that comprises a current component model for classifying the cardiac EGM data as evidence of that respective one of the plurality of cardiac event types; and generate for display output data indicative of a positive detection of the cardiac event type.

In another example, a second medical system configured to run a remote monitoring service for a plurality of medical devices comprises: communication circuitry configured to exchange data with the medical devices corresponding to the remote monitoring service, wherein a portion of the data corresponds to a modular machine learning architecture that the medical devices employ to classify cardiac EGM data as evidence of an arrhythmia episode corresponding to one or more of a plurality of cardiac arrhythmia types; and processing circuitry operative to execute logic for the remote monitoring service, wherein the logic is configured to: maintain, in a storage device, a most recent version of the modular machine learning architecture, wherein the modular machine learning architecture comprises, in each module, a neural network ensemble comprising a current neural network for predicting a likelihood that any given cardiac EGM sample indicates a respective cardiac arrhythmia type and a masked neural network for each other cardiac arrhythmia type; and in response to an update to the modular machine learning architecture, deploy, to the medical devices via the communication circuitry, a new module that corresponds to the update.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF THE DRAWINGS

FIG. 1 illustrates example environment of an example medical system in conjunction with a patient, in accordance with one or more examples of the present disclosure.
FIG. 2 is a functional block diagram illustrating an example configuration of a medical device, in accordance with one or more examples of the present disclosure.
FIG. 3 is a conceptual side-view diagram illustrating an example configuration of the IMD of FIGS. 1 and 2, in accordance with one or more examples of the present disclosure.
FIG. 4 is a functional block diagram illustrating an example configuration of the external device of FIG. 1, in accordance with one or more examples of the present disclosure.
FIG. 5 is a block diagram illustrating an example system that includes an access point, a network, external computing devices, such as a server, and one or more other computing devices, which may be coupled to the medical device and external device of FIGS. 1-4, in accordance with one or more examples of the present disclosure.
FIG. 6 is a flow diagram illustrating an example operation for determining changes in patient health or enabling accurate detection of changes in patient health, in accordance with one or more examples of the present disclosure.
FIG. 7 is a flow diagram illustrating an example operation for updating a modular machine learning architecture for medical devices, in accordance with one or more examples of the present disclosure.
FIGS. 8A-B are both illustrations of a cardio electrogram, in accordance with one or more examples of the present disclosure. FIG. 8A illustrates an example cardiac EGM having a pause episode as an example cardiac event. FIG. 9B illustrates an example cardio electrogram having no cardiac events.
FIG. 9 is a conceptual view of a modular machine learning architecture, in accordance with one or more examples of the present disclosure.
FIGS. 10A-D are conceptual views of modules forming at least part of a modular machine learning architecture, in accordance with one or more examples of the present disclosure.
FIG. 11 includes illustrations of graphs depicting tests of machine learning models, in accordance with one or more examples of the present disclosure.
FIGS. 12A-B are both illustrations of tests and on an ensemble configured to classify a particular cardiac event.

Like reference characters denote like elements throughout the description and figures.

### DETAILED DESCRIPTION

In general, medical systems according to the present disclosure implement techniques for detecting changes in a patient's health (e.g., heart health) based upon data describing the patient's physiology including the patient's cardiac physiology. Cardiac events (e.g., arrhythmias) and other maladies negatively affect heart health in general and may be potentially fatal to the patient. An example medical device captures signals representing the patient's heart's electrical activity and generates cardiac activity data to monitor, evaluate, and then, classify as a cardiac event type. The example medical device executes a machine learning algorithm described herein to generate a prediction value indicating whether or not the patient's cardiac activity data depicts a particular cardiac event type; for instance, the example algorithm may instruct the example medical device to apply a modular machine learning architecture to the cardiac activity data and predict a likelihood of an occurrence of a particular type of cardiac event (e.g., an episode of arrhythmia).

Logical circuitry or circuitries (and, possibly, other hardware) enable the example medical systems and techniques described herein including the above medical device that collects and evaluates the patient's cardiac activity data. Examples of the medical device include wearable devices, implantable devices, and/or any other medical device in communication with one or more sensors (e.g., an electrode) continuously measuring the patient's heart's electrical activities and recording such measurements as the patient's cardiac activity data.

An example computing system may be configured with programmable logic configured to perform an algorithm (e.g., a machine learning algorithm) and may include processing circuitry that, by executing the programmable logic, performs operations in accordance with such an algorithm. Machine learning generally refers to algorithms (e.g., learning) and structures (e.g., machine learning models) enabling functionality (e.g., classification) for making predictions or automating decisions without user direction or explicit rules, which may or may not be hard-coded or otherwise programmed to do so. As instructed by an example machine learning algorithm, the computing system may build a model based on foundational concepts and some training data (e.g., samples of sensor data including patient cardiac EGM data or ECG data), test that model using additional training data and validation data and various metrics, and if the model satisfies the metrics, deploy the model to a medical system that is configured to monitor the patient's cardiac physiology for cardiac events. The medical device may upload samples of cardiac EGM data or ECG data to serve as additional training data for testing models.

The medical systems and devices described herein include techniques to detect changes in patient health based on classifications of the patient's cardiac activity data in accordance with a modular machine learning architecture. Machine learning not only simplifies detection logic in general, by leveraging the modular machine learning architecture, machine learning concepts employed herein accomplish complementary goals of lowering operational resource requirements and lowering overall resource utilization. Conserving resource capacities also results in time and capital savings. Having a lower resource footprint enables smaller and less complex embodiments of these techniques (e.g., implantable or wearable embodiments). A medical device equipped with substantial resource capacities is no longer needed; instead, a medical device with fewer capacities of processing, network, and storage resources can be configured to detect changes in patient health in accordance with any technique described herein.

Medical device manufacturers and/or service providers of a remote monitoring service may avail the modular machine learning architecture to lower a validation burden. As described herein, the modular approach behind the solution to classifying cardiac activity data allows one module to be updated while other modules are unaffected by the update. Because each module is an independent function that is run in parallel with other modules to effectuate the classification of cardiac activity data as types of cardiac events, both the monitoring service and the client medical device benefit from fewer compute and/or storage tasks. Each module may include an ensemble of component models where each component model is configured to classify a respective one of the cardiac event types but the corresponding module's outputs only one component model's classification (e.g., masking classifications by other component model in the same module). The medical device manufacturers and/or service providers of the remote monitoring service may leverage model ensembling to ensure that each module is dedicated to classifying a different cardiac event type and remains unaffected by an update to another module's ensemble. Model ensembling typically improves performance over a single model (e.g., a single neural network).

In view of the above, the present disclosure describes a technological improvement in a technical field or a technical solution that is integrated into a practical application. Medical systems and devices benefit from reductions in false determinations and an improved accuracy of arrhythmia detections. The modular machine learning architecture advantageously permits debugging where parts (e.g., neural network layers) of a module can be replaced, removed, or upgraded while other modules remain unchanged. In this manner, given an example modular architecture where each module includes current versions of multiple component models for predicting different cardiac event types, when a new/more recent version of a component model for predicting a specific cardiac event becomes available (e.g., after sufficient testing), other component models do not need to be tested. Because of interactivity between the multiple component models, the other component models in the same example modular machine learning architecture are typically tested along with the entire module (e.g., as part of the new/more recent model's validation). Instead of validating each cardiac event type's corresponding component model after updating one component model, configuring the new/more recent component model in a separate (e.g., dedicated) module eliminates the validation requirement for the other component models. The other component models have already been validated and therefore, do need repetitive testing neither individually nor as a combination (e.g., an ensemble). The example modular machine learning architecture allows for only validated component models to factor in the final prediction. In addition, individual modules can be changed without requiring regulatory evaluation for the entire architecture, which benefits the patient with the medical device, the medical device manufacturer, and service providers of the remote monitoring service.

The remote monitoring service may run tests on different configurations of the modular machine learning architecture for potential deployment for monitoring a patient or population of patients, e.g., via incorporation into the medical devices. The service provider of the remote monitoring service leverages a more accurate performance metric to evaluate models as potential updates to the architecture. For example, the service provider may use a geometric mean of specificity and sensitivity metric values as the more accurate performance metric when comparing models (e.g., neural networks) to determine which performs better. When necessary, the service provider may use the remote monitoring service to deploy new models as updates to the modular machine learning architecture, e.g., in use at the medical devices.

In addition, the modular machine learning architecture may employ any model type (e.g., artificial neural network ensemble) that can trained/validated using cardiac activity data for a plurality of patients and then, configured to determine a predication value (e.g., a probability) such as a likelihood that (e.g., a sample of) the cardiac activity data constitutes evidence of a cardiac event type.

An example computing system (e.g., a cloud-based system) runs the remote monitoring service to support the example medical devices with data and/or application services. The example computing system may build, test, and then, deploy updates to the modular machine learning architecture in operation at the example medical devices. For instance, the example computing system may train a current machine learning model (e.g., a current neural network) using new training data and then, build a new model (e.g., a new neural network). Due to the modular approach of the machine learning algorithm, the example computing device is only required to communicate changed portion(s) of the model. In some cases, the example computing device communicates an update storing an additional branch to an existing neural network or a replacement branch for an existing branch in that existing neural network. The example medical devices may communicate positive classifications and associated cardiac EGM data samples to device(s) of the example computing system for the remote monitoring service to use for training and testing potential updates to the modular machine learning architecture. One device of the example computing system may also analyze the positive classifications and then, provide a report regarding the patient's cardiac activities and heart health. The report may compare various implementations of the techniques described herein, for example, evaluating different models (e.g., neural networks) using various metrics. Such a report may provide a patient or caregiver information an important aspect of the patient's health.

In this manner, the techniques of this disclosure may advantageously enable improved accuracy in the detection of changes in patient health and, consequently, better evaluation of the condition of the patient.

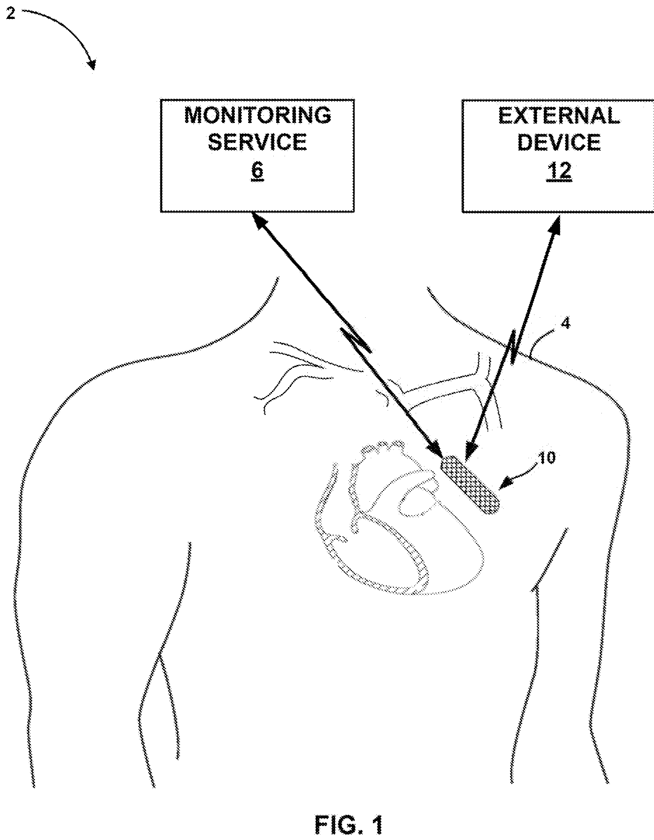

FIG. 1 illustrates the environment of an example medical system 2 in conjunction with a patient 4, in accordance with one or more techniques of this disclosure. It should be noted that in example medical system 2, any computing device may execute the example techniques of the present disclosure. The present disclosure envisions a number of different devices for other examples/alternatives of example medical system 2, and any of these devices can substitute for IMD 10.

IMD 10, which may be in wired/wireless communication with an external device 12 and in communication with at least one device of monitoring service 6 and (possibly) other devices not pictured in FIG. 1, represents any implantable monitoring device for example medical system 2, and the environment illustrated in FIG. 1 includes IMD 10 to demonstrate specific benefits and advantages of the present disclosure. It should be further noted that other devices in the environment illustrated in FIG. 1 may be used with any computer device configured to execute techniques for detecting changes in patient health including the example techniques of this disclosure for detecting occurrences of cardiac events in patient 4.

In one example, IMD 10 implements an example technique directed to detecting cardiac events and/or other heart maladies based on patient 4's cardiac activity. IMD 10 may be a type of cardiac monitoring device, such as a pacemaker/defibrillator or a ventricular assist device (VAD). Implantable medical devices such as IMD 10 may record cardiac activity data for patient 4 in the form of a cardiac EGM. As an alternative, a wearable monitoring device may be configured to collect and evaluate patient 4's cardiac activity data (e.g., cardiac ECG data) in accordance with the present disclosure. Example medical system 2 may be adapted for other devices that are capable of monitoring patient 4's cardiac activity, including smart/personal EKG devices and ECG devices, surface ECG devices, and other heart monitoring devices.

In some examples, IMD 10 is implanted outside of a thoracic cavity of patient 4 (e.g., subcutaneously in the pectoral location illustrated in FIG. 1). IMD 10 may be positioned near the sternum near or just below the level of the heart of patient 4, e.g., at least partially within the cardiac silhouette. IMD 10 includes a plurality of electrodes (not shown in FIG. 1), and is configured to sense electrical activity of the heart via the plurality of electrodes. The sensed electrical activity may be herein referred to as an electrocardiogram (ECG) or a cardiac electrogram (EGM). In some examples, IMD 10 takes the form of the LINQ^{™} ICM available from Medtronic, Inc. of Minneapolis, MN.

It also is possible for example medical system 2 to configure portable computing devices (e.g., mobile/smart phones and tables) into operating as cardiac monitors for patient 4. A mobile device may run a software application to record cardiac activity and then, evaluate that activity for cardiac events.

Monitoring service 6 herein refers to a remote monitoring service that provides, over a network, IMD 10 and/or external device 12 with data and/or application services. In some examples of monitoring service 6, a cloud service provider maintains a set of computing devices operating as data/application servers that are configured to support IMD 10 functionality, for instance, with updates to a machine learning architecture in use by IMD 10. Monitoring service 6 may deploy, as an example update, a new model (e.g., neural network) to add to the machine learning architecture as (e.g., a replacement of ) a current version of a model that is dedicated to classifying cardiac events of a certain type. In some examples, monitoring service 6 may provide a new portion of an existing model, such as new branch of a neural network.

As another benefit to patient 4, the machine learning architecture and the techniques described herein enable efficient hardware resource utilization by IMD 10. In one example, IMD 10 may make efficient use of battery power and memory space by recording more cardiac activity data of actual cardiac events instead. IMD 10 may leverage the machine learning architecture to detect cardiac event earlier and/or avoid recording the cardiac activity of false positives. In order to save battery, some medical devices (e.g., implantable devices) only record first 30 sec and last 30 sec of cardiac activity data (e.g., cardiac ECG data), leaving out a considerable amount of the patient's cardiac activity data from the evaluation. The missing time period may be referred to as a suspension and some devices have substantial suspension periods. Some medical devices have an hour long suspension period where valuable information is not retrievable for review. For lethal cardiac maladies such as Ventricular Tachycardia (VT), doctors want to review the cardiac activity data within the hour long suspension period.

IMD 10 may use monitoring service 6 to make efficient use of battery power and memory space. Monitoring service 6 may enable IMD 10 to record more cardiac activity data of actual cardiac events by confirming or rejecting initial detections of cardiac events. For instance, monitoring service 6 may confirm or reject an arrhythmia determination (e.g., a classification) by IMD 10 regarding patient 4's cardiac activity and IMD 10 may use that confirmation or rejection to determine how much of patient 4's cardiac activity to record. If the arrhythmia detection is confirmed according to the machine learning architecture, monitoring service 6 may instruct IMD 10 to record more cardiac activity of patient 4.

External device 12 may be a computing device with a display (e.g., an electronic display) viewable by the user and an interface for receiving user input to external device 12. In some examples, external device 12 may be a notebook computer, tablet computer, workstation, one or more servers, cellular phone, personal digital assistant, or another computing device that may run an application that enables the computing device to interact with IMD 10. In some examples, external device 12 is a server of monitoring service 6.

External device 12 is configured to communicate with a computing device of monitoring service 6, IMD 10, and, optionally, another computing device (not illustrated in FIG. 1), via wireless communication. External device 12, for example, may communicate via near-field communication technologies (e.g., inductive coupling, NFC or other communication technologies operable at ranges less than 10-20 cm) and far-field communication technologies (e.g., radiofrequency (RF) telemetry according to the 802.11 or Bluetooth^{®} specification sets, or other communication technologies operable at ranges greater than near-field communication technologies).

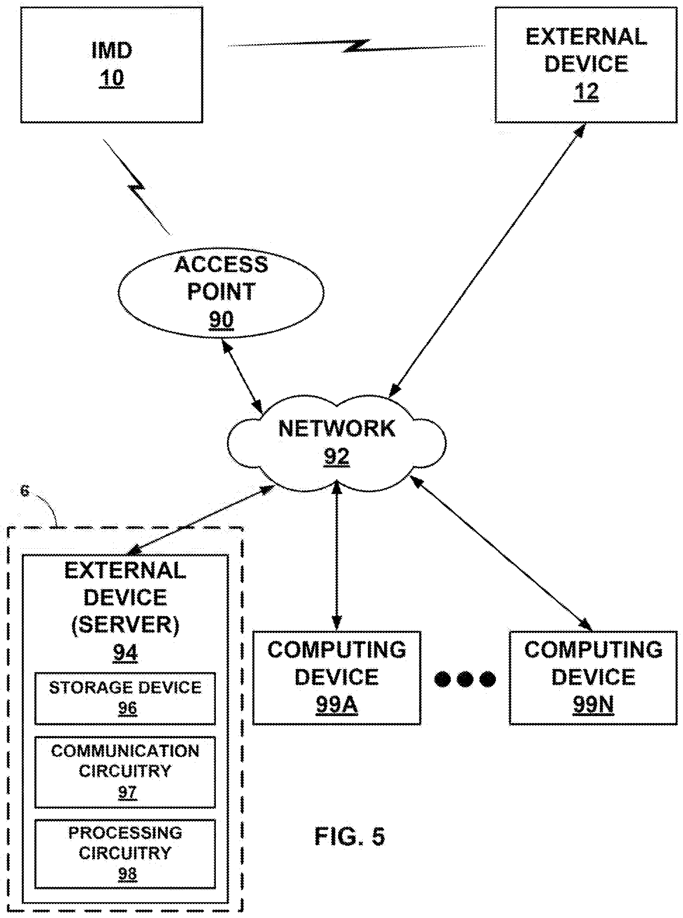

External device 12 may be used to configure operational parameters for IMD 10. External device 12 may be used to retrieve data from IMD 10. The retrieved data may include values of physiological parameters measured by IMD 10, indications of episodes of arrhythmia or other maladies detected by IMD 10, and physiological signals recorded by IMD 10. For example, external device 12 may retrieve cardiac EGM segments recorded by IMD 10 due to IMD 10 determining that an episode of asystole or another malady occurred during the segment. As another example, external device 12 may receive activity data, daily activity metric values, or other data related to the techniques described herein from IMD 10. As will be discussed in greater detail below with respect to FIG. 5, one or more remote computing devices may interact with IMD 10 in a manner similar to external device 12, e.g., to program IMD 10 and/or retrieve data from IMD 10, via a network.

Processing circuitry of medical system 2, e.g., of IMD 10, external device 12, and/or of one or more other computing devices of monitoring service 6, may be configured to perform techniques for detecting changes in patient health including the example techniques of this disclosure for detecting occurrences of cardiac events in patient 4. Processing circuitry of IMD 10 may be communicably coupled to one or more sensors, each being configured to sense physiological parameters in some form (e.g., cardiac physiology parameter(s) corresponding to electrical activity of patient 4's heart), and sensing circuitry configured to generate patient physiological data based on the sensed physiological parameters (e.g., data corresponding to the sensed electrical activity). In some examples, processing circuitry of IMD 10 and/or processing circuitry of external device 12, may execute logic to detect the above-mentioned cardiac events (e.g., arrhythmias) where that logic is configured to perform an algorithm in accordance with the machine learning architecture provided by monitoring service 6.

The machine learning architecture in use by system 2 may be modular and therefore, herein referred to as the modular machine learning architecture. The present disclosure, by "modular", refers to a modular approach to the classification of data corresponding to the sensed electrical activity of the heart of patient 4 (e.g., cardiac EGM data). In such an approach, independent functions (known as modules) provide prediction values for different classes where each class is a cardiac event type (such as an atrial arrhythmia), and each prediction value is uninfluenced by any other independent function. Processing circuitry of system 2, e.g., of IMD 10, executes an example algorithm in accordance with the modular machine learning architecture by applying each module to the data corresponding to the sensed electrical activity and producing a prediction value (e.g., probability) indicative of a likelihood for each cardiac event type. Processing circuitry of system 2, optionally, applies false detection criteria configured to detect one or more indicators of noise and/or amplitude variations in the sensed electrical activity.

Although described in the context of examples in which IMD 10 that senses at least some of the patient physiological parameter data may comprise an insertable cardiac monitor, example systems including one or more implantable, wearable, or external devices of any type configured to sense one or more patient physiological parameter(s) including cardiac physiological parameter(s) corresponding to the electrical activity of the heart of patient 4 may be configured to implement the techniques of this disclosure.

In some examples, processing circuitry in a wearable device may execute same or similar logic as the logic executed by processing circuitry of IMD 10 and/or other processing circuitry as described herein. In this manner, a wearable device or other device may perform some or all of the techniques described herein in the same manner described herein with respect to IMD 10. In some examples, the wearable device operates with IMD 10 and/or external device 12 as potential providers of computing/storage resources and sensors for monitoring patient 4's cardiac physiology including electrical activity and other parameters. For example, the wearable device may communicate the cardiac EGM data to external device 12 for storage in non-volatile memory and for computing prediction values in accordance with the modular machine learning architecture described herein. Similar to processing circuitry of IMD 10, processing circuitry of external device 12 may determine whether the prediction values indicates an occurrence of a particular cardiac event type.

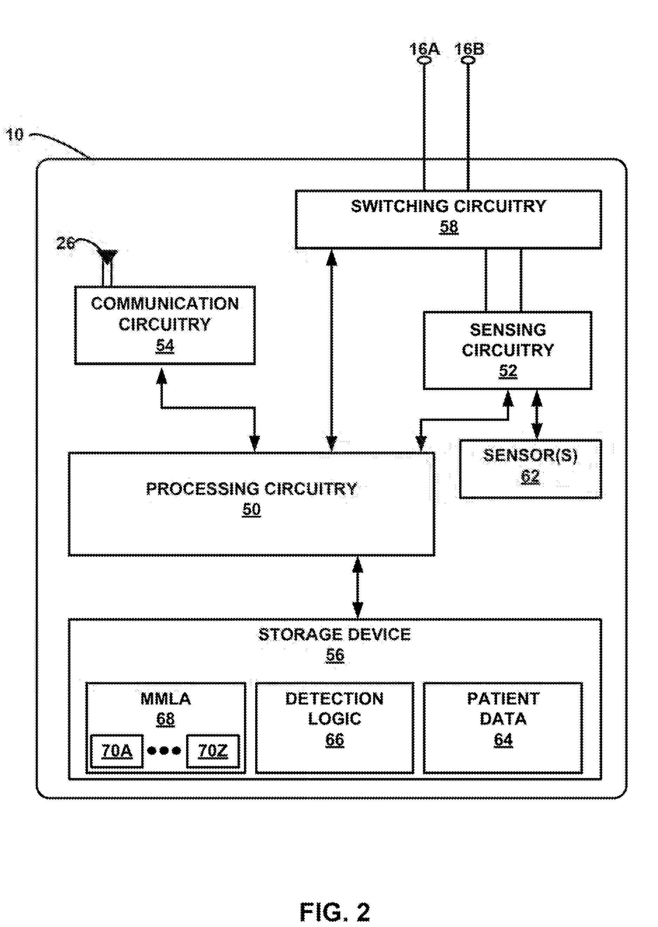

FIG. 2 is a functional block diagram illustrating an example configuration of IMD 10 of FIG. 1 in accordance with one or more techniques described herein. In the illustrated example, IMD 10 includes electrodes 16A and 16B (collectively "electrodes 16"), antenna 26, processing circuitry 50, sensing circuitry 52, communication circuitry 54, storage device 56, switching circuitry 58, and sensors 62. Although the illustrated example includes two electrodes 16, IMDs including or coupled to more than two electrodes 16 may implement the techniques of this disclosure in some examples.

Processing circuitry 50 may include fixed function circuitry and/or programmable processing circuitry. Processing circuitry 50 may include any one or more of a microprocessor, a controller, a digital signal processor (DSP), an application specific integrated circuit (ASIC), a field-programmable gate array (FPGA), or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware or any combination thereof.

Sensing circuitry 52 may generate sensor data from sensor signals received from sensor(s) 62 that encode patient physiological parameters. Sensing circuitry 52 and processing circuitry 50 may store the sensor data as a portion of patient data 64 in storage device 56.

Sensing circuitry 52 may be selectively coupled to electrodes 16 via switching circuitry 58, e.g., to sense electrical signals of the heart of patient 4, for example by selecting the electrodes 16 and polarity, referred to as the sensing vector, used to sense a cardiac EGM, as controlled by processing circuitry 50. Sensing circuitry 52 may sense signals from electrodes 16, e.g., to produce an internal cardiac electrogram (EGM)), in order to facilitate monitoring the electrical activity of the heart. Sensing circuitry 52 may monitor signals from sensors 62, which may include one or more accelerometers, pressure sensors, and/or optical sensors, as examples. In some examples, sensing circuitry 52 may include one or more filters and amplifiers for filtering and amplifying signals received from electrodes 16 and/or sensors 62. Sensing circuitry 52 may capture signals from any one of sensors 62, e.g., to produce patient data 64, in order to facilitate monitoring the electrical activity of the heart and detecting changes in patient health.

Processing circuitry 50, executing detection logic 66 configured to perform a detection analysis for cardiac events, including arrhythmias, that are likely to cause a change (e.g., a decline) in patient health or, otherwise, negatively affect the patient. Processing circuitry 50 may control sensing circuitry 52 to sense cardiac physiology of the patient in some form, e.g., by sensing electrical activity of the patient's heart via a plurality of electrodes, such as electrodes 16. As described herein, the detection analysis is performed on patient data 64 and any other sensor data and, in the context of FIG. 2, data corresponding to the sensed electrical activity (e.g., cardiac electrogram (EGM) data) is stored as patient data 64. Sensing circuitry 52 converts to digital form signals corresponding to the sensed electrical activity and provides the digitized signals to processing circuitry 50 for the detection analysis. Patient data 64 stores the data corresponding to the sensed electrical activity (e.g., the cardiac EGM data) encompassing a period of time (e.g., during which a cardiac episode may have occurred) and, in some examples, includes a sequence of values representing waveforms (e.g., EGM waves, ECG waves, and/or ECG-type waveforms) of a cardiac rhythm. It should be noted that the data corresponding to typical sensed electrical activity records a series of samples representing points on waves (e.g., the P wave, Q wave, R wave, S wave, T wave and U wave), intervals (e.g., PR interval, QRS interval (also called QRS duration), QT interval or RR interval), segments (e.g., PR segment, ST segment or TP segment), complex(es) (e.g., QRS complex), and other components.

In this example, processing circuitry 50 is configured to evaluate cardiac EGM samples (or portions thereof) and/or other sensed signals from any pair of electrodes connected to the patient's heart. Processing circuitry 50 may apply a pattern recognition technique to interpret electrical vectors recorded in corresponding cardiac EGM data as one or more of the above components. In some examples, the waveform may indicate an initial detection of a cardiac event. Then, processing circuitry 50 is configured to apply modular machine learning architecture 68 to the waveform, and based on prediction values of the modular machine learning architecture, determine whether the waveform indicates a cardiac event type.

To detect the cardiac event type for the patient, processing circuitry 50 determines a classification of the cardiac EGM data in accordance with modular machine learning architecture 68 and that classification is the most likely cardiac event type that occurred. In some examples, modular machine learning architecture 68 includes, for each of a plurality of cardiac event types, an ensemble that comprises a current component model for classifying the cardiac EGM data as evidence of that respective one of the plurality of cardiac event types. Processing circuitry 50 generates for display output data indicative of a particular cardiac event type as the classification.

Processing circuitry 50 of IMD 10 may execute logic 66 programmed with modular machine learning architecture 68; while under the control of the executed logic 66, processing circuitry 50 of IMD 10 applies modules 70A-Z where each module is an independent function and part of a solution to determining which cardiac event type most likely occurred or is occurring. In some configurations of modular machine learning architecture 68, each module 70 is dedicated to classification of a respective one of a plurality of cardiac event types and is configured with the above ensemble with only the component model for the respective cardiac event type unmasked. As described herein, in some example embodiments of modular machine learning architecture 68, modules 70A-Z correspond to classifying respective cardiac arrhythmia types and, in each module 70, a neural network ensemble includes a component neural network for predicting a likelihood of each cardiac arrhythmia type. Each component neural network of that ensemble may be configured to classify the cardiac EGM data as a respective one of atrial fibrillation, atrial flutter, atrial flutter and atrial fibrillation, atrioventricular block, intraventricular conduction delay, premature contraction, premature ventricular contraction, premature atrial or atrioventricular junctional contraction, asystole/pause sinus bradycardia, sinus rhythm, sinus tachycardia, supraventricular tachycardia, and ventricular fibrillation but only one component neural network's predication value is used for the classification.

For each of modules 70A-Z such as module 70N, processing circuitry 50 may apply each of the component neural networks to the cardiac EGM data to determine respective values for a plurality of cardiac arrhythmia types, retain for storage the respective value of the arrhythmia type corresponding to module 70N, and discard other values. In some examples, processing circuitry 50 may generate, based on only a respective value for the corresponding cardiac arrhythmia type of that module, data indicative of a prediction of the corresponding cardiac arrhythmia type. Processing circuitry 50 may combine predictions from the ensembles of modules 70A-Z to determine whether to classify the cardiac EGM data as a cardiac arrhythmia type. In response to a detection of a true positive arrhythmia type, processing circuitry 50 generates, for display, output data such as the waveform(s) spanning an occurrence of the cardiac arrhythmia type.

Processing circuitry 50 may employ communication circuitry 54 to communicate with a remote monitoring service such as monitoring service 6 of which external device 12 may or may not be a server. Processing circuitry 50 may communicate a request to the remote monitoring service to verify that modular machine learning architecture 68 is a most recent architecture and that the ensemble of each module 70 includes a current version of a component neural network configured to predict the corresponding arrhythmia type of that module.

Monitoring service 6, via communication circuitry 54, maintains the most recent modular machine learning architecture 68 by deploying updates to client medical devices including IMD 10. Processing circuitry 50 may be configured to receive, from the remote monitoring service, an update to the above-mentioned machine learning ensembles where the update comprises a new branch of a new component model, the new component model, or a new module with the new component model. Example updates, at most, include a new module 70 to install into modular machine learning architecture 68. This is (in part) because of the modular approach which separates the classification of cardiac EGM data into parallel solutions (i.e., modules 70A-Z) and because each module 70 includes an ensemble to mask all but one component model's prediction value and determines the appropriate classification based on only that prediction value. In response to an update, processing circuitry 50 is configured to update one or more machine learning ensembles of modules 70A-Z by one or more of: removing at least a portion of a component model from the ensemble, modifying the at least a portion of a component model, replacing the at least a portion of a component model, adding a new component model to the component models of the ensemble, or adding a new branch to one of the component models.

Communication circuitry 54 may include any suitable hardware, firmware, software or any combination thereof for communicating with another device, such as external device 12, another networked computing device, or another IMD or sensor. Under the control of processing circuitry 50, communication circuitry 54 may receive downlink telemetry from, as well as send uplink telemetry to external device 12 or another device with the aid of an internal or external antenna, e.g., antenna 26. In addition, processing circuitry 50 may communicate with a networked computing device via an external device (e.g., external device 12) and a computer network, such as the Medtronic CareLink^{®} Network. Antenna 26 and communication circuitry 54 may be configured to transmit and/or receive signals via inductive coupling, electromagnetic coupling, Near Field Communication (NFC), Radio Frequency (RF) communication, Bluetooth, WiFi, or other proprietary or non-proprietary wireless communication schemes.

In some examples, storage device 56 includes computer-readable instructions that, when executed by processing circuitry 50, cause IMD 10 and processing circuitry 50 to perform various functions attributed to IMD 10 and processing circuitry 50 herein. Storage device 56 may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Storage device 56 may store, as examples, programmed values for one or more operational parameters of IMD 10 and/or data collected by IMD 10 for transmission to another device using communication circuitry 54. Data stored by storage device 56 and transmitted by communication circuitry 54 to one or more other devices may include patient cardiac EGM data and other patient data 64 for indications of suspected changes in patient health.

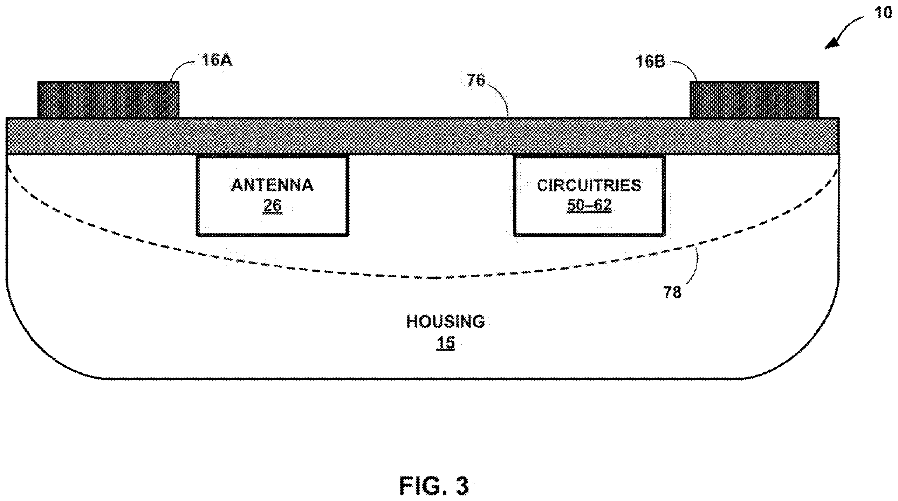

FIG. 3 is a conceptual side-view diagram illustrating an example configuration of IMD 10 of FIGS. 1 and 2. While different examples of IMD 10 may include leads, in the example shown in FIG. 3, IMD 10 may include a leadless, subcutaneously-implantable monitoring device having a housing 15 and an insulative cover 76. Electrode 16A and electrode 16B may be formed or placed on an outer surface of cover 76. Circuitries 50-62, described above with respect to FIG. 2, may be formed or placed on an inner surface of cover 76, or within housing 15. In the illustrated example, antenna 26 is formed or placed on the inner surface of cover 76, but may be formed or placed on the outer surface in some examples. In some examples, insulative cover 76 may be positioned over an open housing 15 such that housing 15 and cover 76 enclose antenna 26 and circuitries 50-62, and protect the antenna and circuitries from fluids such as body fluids.

One or more of antenna 26 or circuitries 50-62 may be formed on the inner side of insulative cover 76, such as by using flip-chip technology. Insulative cover 76 may be flipped onto a housing 15. When flipped and placed onto housing 15, the components of IMD 10 formed on the inner side of insulative cover 76 may be positioned in a gap 78 defined by housing 15. Electrodes 16 may be electrically connected to switching circuitry 58 through one or more vias (not shown) formed through insulative cover 76. Insulative cover 76 may be formed of sapphire (i.e., corundum), glass, parylene, and/or any other suitable insulating material. Housing 15 may be formed from titanium or any other suitable material (e.g., a biocompatible material). Electrodes 16 may be formed from any of stainless steel, titanium, platinum, iridium, or alloys thereof. In addition, electrodes 16 may be coated with a material such as titanium nitride or fractal titanium nitride, although other suitable materials and coatings for such electrodes may be used.

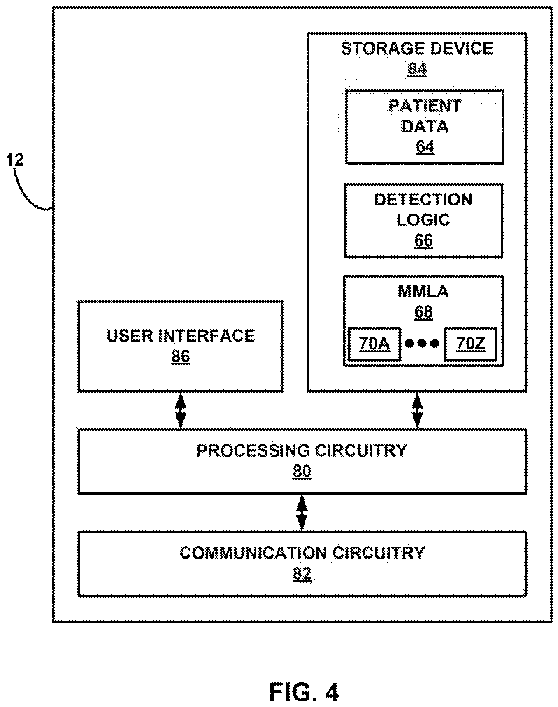

FIG. 4 is a block diagram illustrating an example configuration of components of external device 12. In the example of FIG. 4, external device 12 includes processing circuitry 80, communication circuitry 82, storage device 84, and user interface 86.

Processing circuitry 80 may include one or more processors that are configured to implement functionality and/or process instructions for execution within external device 12. For example, processing circuitry 80 may be capable of processing instructions stored in storage device 84. Processing circuitry 80 may include, for example, microprocessors, DSPs, ASICs, FPGAs, or equivalent discrete or integrated logic circuitry, or a combination of any of the foregoing devices or circuitry. Accordingly, processing circuitry 80 may include any suitable structure, whether in hardware, software, firmware, or any combination thereof, to perform the functions ascribed herein to processing circuitry 80.

Communication circuitry 82 may include any suitable hardware, firmware, software or any combination thereof for communicating with another device, such as IMD 10. Under the control of processing circuitry 80, communication circuitry 82 may receive downlink telemetry from, as well as send uplink telemetry to, IMD 10, or another device. Communication circuitry 82 may be configured to transmit or receive signals via inductive coupling, electromagnetic coupling, NFC, RF communication, Bluetooth, WiFi, or other proprietary or non-proprietary wireless communication schemes. Communication circuitry 82 may also be configured to communicate with devices other than IMD 10 via any of a variety of forms of wired and/or wireless communication and/or network protocols.

Storage device 84 may be configured to store information within external device 12 during operation. Storage device 84 may include a computer-readable storage medium or computer-readable storage device. In some examples, storage device 84 includes one or more of a short-term memory or a long-term memory. Storage device 84 may include, for example, RAM, DRAM, SRAM, magnetic discs, optical discs, flash memories, or forms of EPROM or EEPROM. In some examples, storage device 84 is used to store data indicative of instructions for execution by processing circuitry 80. Storage device 84 may be used by software or applications running on external device 12 to temporarily store information during program execution.

Data exchanged between external device 12 and IMD 10 may include operational parameters. External device 12 may transmit data including computer readable instructions which, when implemented by IMD 10, may control IMD 10 to change one or more operational parameters and/or export collected data. Processing circuitry 80 may also transmit an instruction to IMD 10 which requests IMD 10 to export collected data to external device 12, or IMD 10 may export collected data to external device 12 opportunistically or according to a schedule. In turn, external device 12 may receive the collected data from IMD 10 and store the collected data in storage device 84. The data external device 12 receives from IMD 10 may include patient data 64 including data indicative of patient physiological parameters and/or episode data indicative of cardiac events that most likely occurred in the patient. In one example, IMD 10 may generate sensor data from captured signals and forward that sensor data to external device 12 for storage in patient data 64 with other sensor data for a same patient. Processing circuitry 80 may implement any of the techniques described herein to analyze cardiac activity data from IMD 10 to determine whether the cardiac activity data constitutes as evidence of a cardiac event that occurred in the patient at some point in time e.g., to determine whether the patient is experiencing a change in health.

A user, such as a clinician or patient 4, may interact with external device 12 through user interface 86. User interface 86 includes a display (not shown), such as a liquid crystal display (LCD) or a light emitting diode (LED) display or other type of screen, with which processing circuitry 80 may present information related to IMD 10, e.g., patient cardiac activity data, an indication of a cardiac event based on the patient cardiac activity data, prediction values of potential classes (e.g., types) of cardiac events, a classification of the patient cardiac activity data as indicative of a cardiac event type based on the prediction values, and indications of changes in patient health that correlated to the classification. In addition, user interface 86 may include an input mechanism configured to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 80 of external device 12 and provide input. In other examples, user interface 86 also includes audio circuitry for providing audible notifications, instructions or other sounds to the user, receiving voice commands from the user, or both.

Processing circuitry 80 executes detection logic 66 configured to perform a detection analysis to detect any change (e.g., a decline) in patient health. Processing circuitry 80 may apply the detection analysis to various data corresponding to the sensed electrical activity stored as patient data 64. The detection analysis may be configured to review episode data indicative of a positive cardiac event detection by IMD 10 and, in some examples, confirm or reject the episode data as indicative of that cardiac event. As described herein for FIG. 2, IMD 10, utilizing one or more electrodes for sensing electrical activity of a heart of the patient, includes sensing circuitry to generate data to record digitized signals representing the electrical activity and communication circuity to communicate the generated data to external device 12. Processing circuitry 80 may receive data recording the digitized signals and then, identify one or more electrocardiogram (ECG) or ECG-type waveforms from such data, apply the modular machine learning architecture to the one or more waveforms, and based on prediction values of the modular machine learning architecture, determine whether the one or more waveforms indicate the cardiac event type. In some examples, IMD 10, using any one of a number of cardiac event detection techniques, performs an initial detection analysis and generates, for output, an initial detection of a cardiac event in addition to the data representing the one or more waveforms.

Then, IMD 10 forwards a representation of the one or more waveforms to external device 12 where processing circuitry 80 is to apply modular machine learning architecture 68 and based on prediction values produced by the modular machine learning architecture 68, determine whether the one or more waveforms indicate a cardiac event type. In one example, processing circuitry 80 generates, for output, data indicative of the cardiac event having a highest prediction value that exceeds a threshold value and if processing circuitry 80 determines that the cardiac event matches the cardiac event initially detected by IMD 10, further generates, for output, data indicative of a confirmation of IMD 10's initial detection. In contrast, if processing circuitry 80 determines that the cardiac event does not match the cardiac event initially detected by IMD 10, generates, for output, data indicative of a rejection of IMD 10's initial detection.

To detect the cardiac event type for the patient, processing circuitry 80 determines a classification of the waveform in accordance with modular machine learning architecture 68 and that classification is the most likely cardiac event type that occurred. In some examples, modular machine learning architecture 68 includes, for each of a plurality of cardiac event types, an ensemble that comprises a current component model for classifying the waveform as evidence of that respective one of the plurality of cardiac event types; and generate for display output data indicative of a positive detection of the cardiac event type.

Processing circuitry 80 of IMD 10 may execute logic 66 programmed with modular machine learning architecture 68; while under the control of the executed logic 66, processing circuitry 80 of IMD 10 applies modules 70A-Z where each module is an independent function and part of a solution to determining which cardiac event type most likely occurred or is occurred. In some configurations of modular machine learning architecture 68, each module 70 is dedicated to classification of a respective one of a plurality of cardiac event types and is configured with the above ensemble with only the component model for the respective cardiac event type unmasked. As described herein, in some example embodiments of modular machine learning architecture 68, modules 70A-Z correspond to classifying respective cardiac arrhythmia types and, in each module 70, a neural network ensemble includes a component neural network for predicting a likelihood of each cardiac arrhythmia type. Each component neural network of that ensemble may be configured to classify the cardiac activity data (e.g., sample(s) of cardiac EGM data) as a respective one of atrial fibrillation, atrial flutter, atrial flutter and atrial fibrillation, atrioventricular block, intraventricular conduction delay, premature contraction, premature ventricular contraction, premature atrial or atrioventricular junctional contraction, asystole/pause sinus bradycardia, sinus rhythm, sinus tachycardia, supraventricular tachycardia, and ventricular fibrillation but only one component neural network's predication value is used for the classification. It should be noted that the samples of cardiac EGM data may or may not correspond to a suspected cardiac episode.

For each of modules 70A-Z such as module 70N, processing circuitry 80 may apply each of the component neural networks to the cardiac EGM data to determine respective values for a plurality of cardiac arrhythmia types, retain for storage the respective value of the arrhythmia type corresponding to module 70N, and discard other values. In some examples, processing circuitry 80 may generate, based on only a respective value for the corresponding cardiac arrhythmia type of that module, data indicative of a prediction of the corresponding cardiac arrhythmia type. Processing circuitry 80 may combine predictions from the ensembles of modules 70A-Z to determine whether to classify the cardiac EGM data as a cardiac arrhythmia type. In response to a detection of a true positive arrhythmia type, processing circuitry 80 generates, for display, output data such as the one or more waveforms spanning an occurrence of the cardiac arrhythmia type.

Processing circuitry 80 may employ communication circuitry 54 to communicate with a remote monitoring service such as monitoring service 6 of which external device 12 may or may not be a server. Processing circuitry 80 may communicate a request to the remote monitoring service to verify that modular machine learning architecture 68 is a most recent architecture and that the ensemble of each module 70 includes a current version of a component neural network configured to predict the corresponding arrhythmia type of that module.

Monitoring service 6 configures modular machine learning architecture 68 with up-to-date models for detecting cardiac arrhythmia types. Other machine learning techniques combine the up-to-date models into a single model and must have that single model validated by appropriate government agencies. The present disclosure describes a number of benefits to monitoring service 6 ; as one benefit, monitoring service 6 does not have to wait for validation of the single model and may deploy an up-to-date model for a specific cardiac arrhythmia type as soon as that specific model is validated. Modular machine learning architecture 68 configures each module 70 with a particular up-to-date model for one cardiac arrhythmia type and any (validated) model for each other cardiac arrhythmia type. In this manner, each module 70 includes a validated ensemble model for detecting cardiac arrhythmia types.

Processing circuitry 80 may be configured to deploy, from remote monitoring service 6, an update to the above-mentioned machine learning ensembles where the update comprises a new branch of a new component model, the new component model, or a new module with the new component model. Example updates, at most, include a new module 70 to install into modular machine learning architecture 68. This is (in part) because of the modular approach which separates the classification of cardiac EGM data into parallel solutions (i.e., modules 70A-Z) and because each module 70 includes an ensemble to mask all but one component model's prediction value and determines the appropriate classification based on only that prediction value. In response to an update, processing circuitry 80 is configured to update one or more machine learning ensembles of modules 70A-Z by one or more of: removing at least a portion of a component model from the ensemble, modifying the at least a portion of a component model, replacing the at least a portion of a component model, adding a new component model to the component models of the ensemble, or adding a new branch to one of the component models.

FIG. 5 is a block diagram illustrating an example system that includes an access point 90, a network 92, external computing devices, such as a server 94, and one or more other computing devices 99A-99N (collectively, "computing devices 99"), which may be coupled to IMD 10 and external device 12 via network 92, in accordance with one or more techniques described herein. In this example, IMD 10 may use communication circuitry 54 to communicate with external device 12 via a first wireless connection, and to communicate with an access point 90 via a second wireless connection. In the example of FIG. 5, access point 90, external device 12, server 94, and computing devices 99 are interconnected and may communicate with each other through network 92.

Access point 90 may include a device that connects to network 92 via any of a variety of connections, such as telephone dial-up, digital subscriber line (DSL), or cable modem connections. In other examples, access point 90 may be coupled to network 92 through different forms of connections, including wired or wireless connections. In some examples, access point 90 may be a user device, such as a tablet or smartphone, that may be co-located with the patient. IMD 10 may be configured to transmit data, such as patient data corresponding to the patient's cardiac physiology including electrical activity of the patient's heart and/or indications of changes in patient health, to access point 90. Access point 90 may then communicate the retrieved data to server 94 via network 92.

Server 94, as part of monitoring service 6, provides data and application services to a plurality of medical devices including IMD 10. As described herein, monitoring service 6 supports cardiac event detection by way of machine learning at each medical device. In the example illustrated by FIG. 5, server 94 includes storage device 96, communication circuitry 97, and processing circuitry 98. Server 94 configures communication circuitry 97 to exchange various with the plurality of medical devices and/or external devices 12 data of which a portion corresponds to a modular machine learning architecture that the medical devices, including IMD 10, employ to classify cardiac EGM data as evidence of an arrhythmia episode corresponding to one or more of a plurality of cardiac arrhythmia types. Processing circuitry 98 is operative to execute logic for monitoring service 6, such as logic configured to maintain, in storage device 96, a most recent version of the modular machine learning architecture; in each module, a neural network ensemble includes a current neural network for predicting a likelihood that any given cardiac EGM sample indicates a respective cardiac arrhythmia type and a masked neural network for each other cardiac arrhythmia type. In response to an update to the modular machine learning architecture, processing circuitry 98 deploys, to the medical devices via the communication circuitry, a new module that corresponds to the update.

In some cases, server 94 may be configured to provide a secure storage site for data that has been collected from IMD 10 and/or external device 12. In some cases, server 94 may assemble data in web pages or other documents for viewing by trained professionals, such as clinicians, via computing devices 99. One or more aspects of the illustrated system of FIG. 5 may be implemented with general network technology and functionality, which may be similar to that provided by the Medtronic CareLink^{®} Network.

In some examples, one or more of computing devices 99 may be a tablet or other smart device located with a clinician, by which the clinician may program, receive alerts from, and/or interrogate IMD 10. For example, the clinician may access cardiac EGM data and/or indications of patient health collected by IMD 10 through a computing device 99, such as when patient 4 is in in between clinician visits, to check on a status of a medical condition. In some examples, the clinician may enter instructions for a medical intervention for patient 4 into an application executed by computing device 99, such as based on a status of a patient condition determined by IMD 10, external device 12, server 94, or any combination thereof, or based on other patient data known to the clinician. Device 99 then may transmit the instructions for medical intervention to another of computing devices 99 located with patient 4 or a caregiver of patient 4. For example, such instructions for medical intervention may include an instruction to change a drug dosage, timing, or selection, to schedule a visit with the clinician, or to seek medical attention. In further examples, a computing device 99 may generate an alert to patient 4 based on a status of a medical condition of patient 4, which may enable patient 4 proactively to seek medical attention prior to receiving instructions for a medical intervention. In this manner, patient 4 may be empowered to take action, as needed, to address his or her medical status, which may help improve clinical outcomes for patient 4.

In the example illustrated by FIG. 5, server 94 includes storage device 96, e.g., to store data retrieved from IMD 10, and processing circuitry 98. Although not illustrated in FIG. 5 computing devices 99 may similarly include a storage device and processing circuitry. Processing circuitry 98 may include one or more processors that are configured to implement functionality and/or process instructions for execution within server 94. For example, processing circuitry 98 may be capable of processing instructions stored in storage device 96. Processing circuitry 98 may include, for example, microprocessors, DSPs, ASICs, FPGAs, or equivalent discrete or integrated logic circuitry, or a combination of any of the foregoing devices or circuitry. Accordingly, processing circuitry 98 may include any suitable structure, whether in hardware, software, firmware, or any combination thereof, to perform the functions ascribed herein to processing circuitry 98. Processing circuitry 98 of server 94 and/or the processing circuity of computing devices 99 may implement any of the techniques described herein to analyze information, data, or data 64 received from IMD 10, e.g., to determine whether the health status of a patient has changed, to determine whether prediction criteria are satisfied and/or false prediction criteria are satisfied.

Storage device 96 may include a computer-readable storage medium or computer-readable storage device. In some examples, storage device 96 includes one or more of a short-term memory or a long-term memory. Storage device 96 may include, for example, RAM, DRAM, SRAM, magnetic discs, optical discs, flash memories, or forms of EPROM or EEPROM. In some examples, storage device 96 is used to store data indicative of instructions for execution by processing circuitry 98.

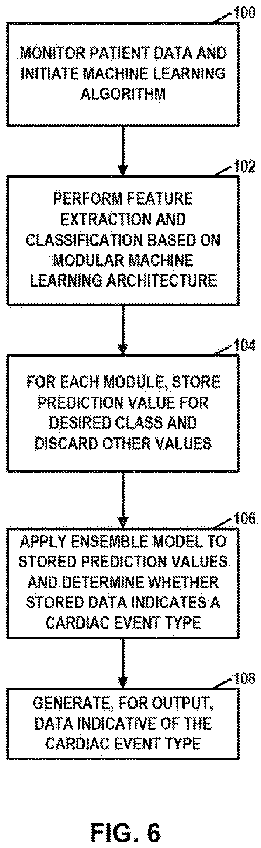

FIG. 6 is a flow diagram illustrating an example operation for determining changes in patient health or enabling accurate detection of changes in patient health, in accordance with one or more examples of the present disclosure. In some examples, the example operation may be implemented for detecting a cardiac event type based on a classification of input sensor data in accordance with a modular machine learning architecture.

In some medical systems, a wearable or implantable medical device or another computing device utilizes the modular machine learning architecture to verify an initial or preliminary arrhythmia detection as a true arrhythmia. Although FIG. 6 is described in the context of an example in which processing circuitry 50 of IMD 10 utilizes the modular machine learning architecture, the example techniques of FIG. 6 may be performed in whole or part by other processing circuitry of system 2, such as processing circuitry 80 of external device 12. Based upon cardiac EGM data corresponding to the initial arrhythmia detection as input, the medical device uses the modular machine learning architecture to output data indicative of a likelihood that the cardiac EGM data is an occurrence of a true arrhythmia for a certain type. Based on recency of the cardiac EGM data, the arrhythmia type may be occurring in a patient.

According to the illustrated example of FIG. 6, processing circuitry 50 of IMD 10 monitors patient data generated by sensing circuitry 52 of IMD 10 (100). For example, as discussed in greater detail with respect to FIGS. 1-5, processing circuitry 50 may monitor patient data (e.g., for a time period) and initiate a machine learning algorithm to determine whether the patient data includes evidence of a particular cardiac event type such as a type of arrhythmia. Some of the patient data includes cardiac EGMs of which each is a representation of physiological parameters corresponding to the patient's cardiac activity including electrical activity of the patient's heart.

In the illustrated example, processing circuitry 50 of IMD 10 performs feature extraction and classification (operations as part of the machine learning algorithm) based on the modular machine learning architecture (102). For each module, processing circuitry 50 of IMD 10 stores a prediction value for a desired output class and discards other prediction values (104). As described herein, each output class corresponds to a type of cardiac event and by discarding all but one unique output class, one module's classification does not affect another module's classification. To illustrate by way of example, if one module is to be updated with a current version of a machine learning model (e.g., a neural network) for a cardiac event type of interest, other modules are not modified because their machine learning models are up-to-date; each other module include a current version of a machine learning model (e.g., a neural network) for that module's corresponding cardiac event type of interest. Monitoring service 6 benefits from such modularity because updates may be developed and then, distributed at a module-level instead of the entire modular machine learning architecture. The modular approach of the example operation allows the medical device to maintain a most recent modular machine learning architecture.

Processing circuitry 50 of IMD 10 applies an ensemble model to stored prediction values and determines whether the stored data indicates a cardiac event of a certain type has occurred or is occurring (106). In some examples, the ensemble model selects a highest prediction value while in other examples, the ensemble module applies various criterion to determining whether a cardiac event occurred and of which cardiac event type is most likely. Processing circuitry 50 of IMD 10 generates, for output, data indicative of the cardiac event type (108).

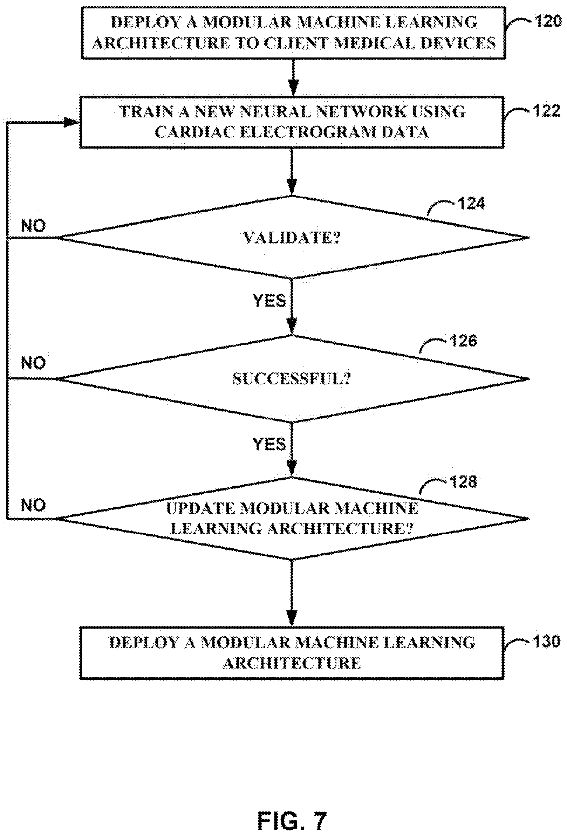

FIG. 7 is a flow diagram illustrating an example operation for updating a modular machine learning architecture for medical devices, in accordance with one or more examples of the present disclosure. Although FIG. 7 is described in the context of an example in which processing circuitry 50 of IMD 10 utilizes the modular machine learning architecture, the example techniques of FIG. 7 may be performed in whole or part by other processing circuitry of system 2, such as processing circuitry 80 of external device 12.

According to the illustrated example of FIG. 7, processing circuitry 98 of server 94 runs a remote monitoring service, such as monitoring service 6, configured to maintain and upgrade a modular machine learning architecture in use by a plurality of medical devices including IMD 10. Processing circuitry 50 of IMD 10 classifies patient data generated by sensing circuitry 52 of IMD 10 as one or more arrhythmia types in accordance with the modular machine learning architecture. After building and testing new models for inclusion in a most recent version of the modular machine learning architecture, processing circuitry 98 of server 94 distributes the new models as updates to the modular machine learning architecture.

In the illustrated example of FIG. 7, processing circuitry 98 of server 94 leverages a communications network to deploy the most recent modular machine learning architecture to client medical devices (120). To commence such a test of a new neural network in the example operation of FIG. 7, processing circuitry 98 of server 94 trains a new neural network using cardiac EGM data (122). Processing circuitry 98 of server 94 determines whether the new neural network has been sufficiently trained and if so, continues testing the neural network. Based on a determination that the new neural network is not sufficiently trained and therefore, is not to be validated by way of the validation data (NO of 124), processing circuitry 98 of server 94 returns to training the new neural network (122).

Based on a determination to use validation data to continue testing the new neural network (YES of 124), processing circuitry 98 of server 94 proceeds to apply the new neural network to the validation data. Processing circuitry 98 of server 94 determines whether the new neural network successfully classified the validation data (126). If the new neural network successfully classified the validation data (YES of 126), processing circuitry 98 of server 94 determined whether to update the modular machine learning architecture in use by the client medical devices (128). Based on the determination that the new neural network did not successfully classify the validation data (NO of 126), processing circuitry 98 of server 94 returns to training the new neural network or another new neural network (122). Processing circuitry 98 of server 94 also returns to training the new neural network or another new neural network (122) based on the determination that the modular machine learning architecture is not to be updated (NO of 128).

Based on a determination to update the modular machine learning architecture after testing new neural network (YES of 128), processing circuitry 98 of server 94 proceeds to deploy the new neural network to the client medical devices (130). In one example, processing circuitry 98 of server 94 may generate a new module that includes the new neural network for classifying a specific arrhythmia type and masked neural networks for classifying other arrhythmia types. An example client medical device, in response to the update, may replace the previous module with the deployed module. Alternatively, an example client medical device, in response to an update comprising the new neural network, may generate a new module in the modular machine learning architecture with the new neural network for classifying a specific arrhythmia type and masked neural networks for other types.

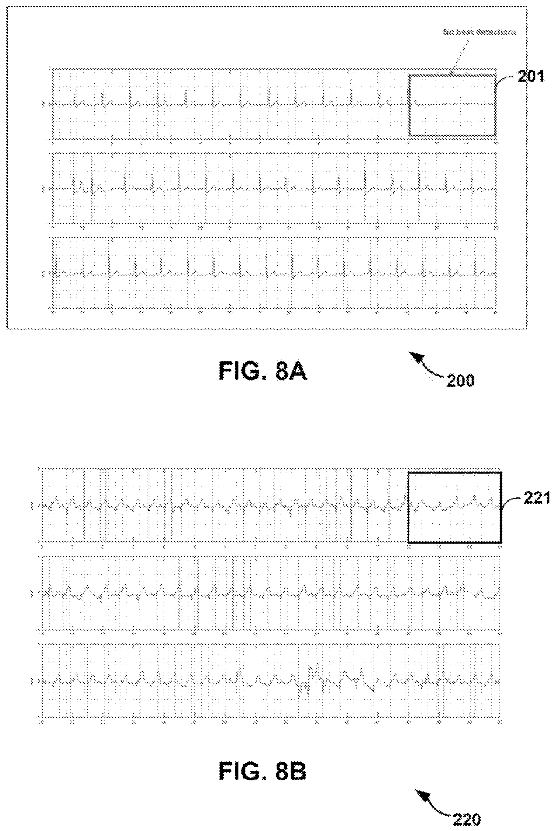

FIGS. 8A-B are both illustrations of cardiac EGMs, in accordance with one or more examples of the present disclosure. FIG. 8A illustrates an example cardiac EGM 200 having a pause episode as an example cardiac event (e.g., arrhythmia). FIG. 8B illustrates an example cardiac EGM 220 having no cardiac events. Both cardiac EGM 200 and cardiac EGM 220 may be partitioned into samples of cardiac EGM data where each sample represents electrical activity of a patient's heart for a period of time. In accordance with a modular machine learning architecture, logic or processing circuitry applies modules to each sample where each module is configured to classify a respective one of a plurality of arrhythmia types.

In FIG. 8A, when applied to sample 201, only the module configured to detect pause episodes produces a positive, non-trivial prediction value. In FIG. 8B, when applied to sample 221, none of the modules produce a positive, non-trivial prediction value because that sample does not indicate an arrhythmia.

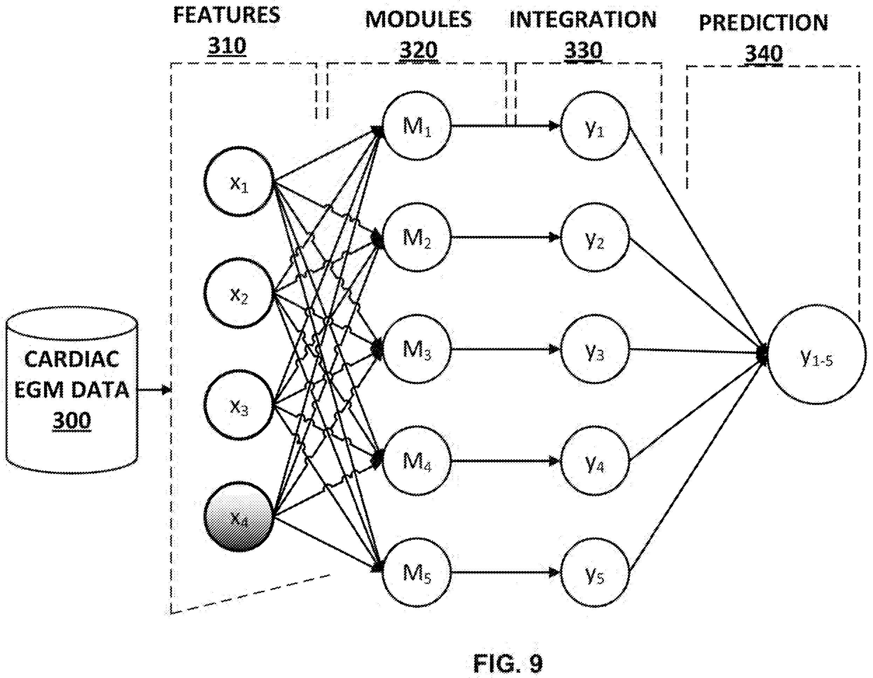

FIG. 9 is a conceptual view of a modular machine learning architecture, in accordance with one or more examples of the present disclosure. With reference to FIGS. 1-5, processing circuitry 50, executing detection logic 66, initiates a machine learning algorithm in response to cardiac EGM data 300 and in accordance with the modular machine learning architecture, classifies samples of the cardiac EGM data 300 as one of a plurality of arrythmia types or no arrhythmia.

For any given sample of cardiac EGM data, processing circuitry 50 is configured to extract features 310 from that sample of cardiac EGM data 300. In accordance with the modular machine learning architecture, processing circuitry 50 applies modules 320 to the sample of cardiac EGM data 300 and each module produces a prediction value for a specific type of arrythmia. At integration 330, processing circuitry 50 evaluates each independent prediction value and determines an appropriate prediction 340.

For each module, processing circuitry 50 applies an ensemble of neural networks (or neural network ensemble or simply ensemble) to features 310 where each component neural network is applied to a copy of features 310. An ensembling technique refers to a model and/or method for combining the component neural networks of each module in some manner to produce the independent prediction values for integration 330. In any given module, one example ensembling technique may mask all but one component neural network's output (intermediary) prediction value while another technique combines component neural network's output (intermediary) prediction values into a prediction value for that given module's corresponding arrythmia type. A module's corresponding arrythmia type refers to the arrythmia type that the module is dedicated to classifying.

Monitoring service 6 or another cloud-based system may improve modular machine learning architecture in use at IMD 10 (or another client medical device) by adding, removing, replacing, or upgraded a module for a particular arrhythmia type. A module may be replaced by a different version without affecting other modules. A module may be upgraded by having a neural network branch upgraded, removed, or replaced. A module may have a branch replaced by a known model or a proprietary model.

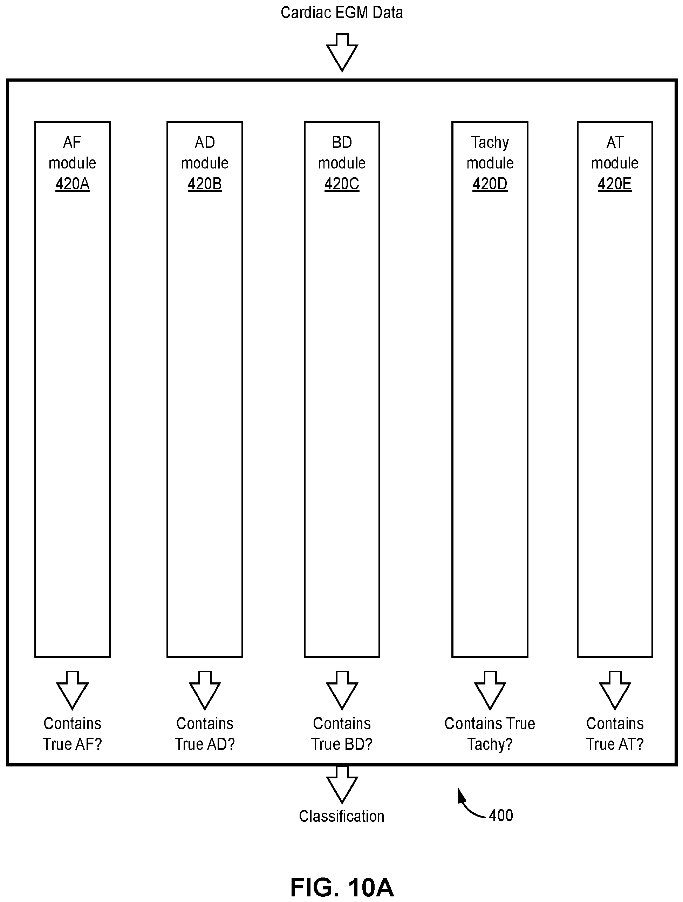

In any given module's ensemble of neural networks, at least one component neural network may also be an ensemble. FIGS. 10A-D illustrate an example of such an architecture. Any known or proprietary ensemble model may be implemented for the at least one component neural network.

Examples of proprietary models include: 1) Dynamic Baseline Subtraction; 2) Dynamic Scaling; and 3) Global Zoom. For a Dynamic Baseline Subtraction Neural Network, an encoder-decoder convolutional neural network may first learn a baseline from cardiac EGM data and then, feed the cardiac EGM-baseline into another neural network to determine the prediction value and make the classification. Such a model solves the baseline wondering problem and improves ensemble accuracy over a single model. Regarding a Dynamic Scaling Neural Network, an encoder-decoder convolutional neural network first learns a scale_vector with equal length to the cardiac EGM data such that each point in the scale_vector is between 0 and 1 and then, feeds vector product (EGM*scale_vector) into another neural network to determine the prediction value and make the classification. This model solved the P wave too small/R wave too big problem and improved ensemble accuracy, for example, by magnifying differences in p-wave morphology . Regarding Global Zoom Neural Network, an encoder-decoder convolutional neural network first learns a zoom_scalar, which is a single number between 0 and 1, and then, feeds clipped_EGM, which is a value derived from clip(EGM, - zoom_scalar, + zoom_scalar), into another neural network to determine the prediction value and make the classification. This model also solved the P-wave too small/R-wave too big problem and improved ensemble accuracy. It should be noted that known models may be known encoder-decoder convolutional neural networks may employed to first learn the cardiac EGM-baseline, the vector product (EGM*scale_vector), and/or the clipped_EGM=clip(EGM, - zoom_scalar, + zoom_scalar) and then, feed one or more of these constructs to an ensembling method or model (e.g., another known neural network) to determine the prediction value and make the classification.

FIGS. 10A-D are conceptual views of modules forming at least part of a modular machine learning architecture, in accordance with one or more examples of the present disclosure.

FIG. 10A is an illustration of an example modular machine learning architecture 400 comprising a plurality of modules 420A-420E (collectively, "modules 420"), where each module is configured to detect a different arrhythmia type and to only output a prediction from a component model (e.g., neural network) for that arrhythmia type. As illustrated in FIG. 10A, in each module, modular machine learning architecture 400 leaves unmasked a current version of a neural network dedicated to classifying a desired arrhythmia type while masking other neural networks. In some examples, modular machine learning architecture 400 updates a module with a new neural network for the classifying the desired arrhythmia type for that module but does not update any other network in that module because, as one reason, only the new neural network will be utilized for the module's classification of cardiac EGM data. FIG. 10A illustrates the above modular approach of modular machine learning architecture 400 by depicting, in each module, only the neural network configured to classify the module's desired arrythmia type. For example, FIG. 10A depicts modular machine learning architecture 400 as having five modules for the arrythmia types: Atrial flutter (AF) module 420A, Pause (AD) module 420B, Brady (BD) module 420C, Tachy module 420D, and atrial fibrillation (AT) module 420E. Each module only outputs a prediction value (e.g., a probability) indicative of a likelihood of that module's desired arrythmia type; for example, the AF module only outputs a Boolean (e.g., a probability of 100%) indicating whether the cardiac EGM data contains a true atrial fibrillation.

In some examples, modular machine learning architecture 400 may be further configured to classify the cardiac EGM data as an arrhythmia type with a specific origin. A tachycardia may originate from left vs right side of the heart and/or from ventricular vs atrial part of the heart. In some examples, modular machine learning architecture 400 may be further configured to determine whether the arrhythmia classification of the cardiac EGM data is monomorphic or polymorphic. Modular machine learning architecture 400 may be further configured to determine a length of time (e.g., 10 or 30 seconds) for the arrhythmia classification, for example, by determining onset and offsets times. For example, a strip of EGM data may be classified as a VT that started at 10.5 second timestamp of the strip and ended at 45.2 second timestamp.

For example, AF module 420A may be a 4-branch neural network ensemble, AD module 420B may include multiple branches, BD module 420C and AT module 420E may both comprise 3-branch neural network ensembles, and Tachy module 420D may be a single branch neural network. When a remote monitoring service (e.g., monitoring service 6) indicates that an update ready for distribution, the remote monitoring service may deploy an update to an individual branch of any of the above modules. As described herein, the remote monitoring service may continue training a current neural network of modular machine learning architecture 400 and to generate a new neural network, the remote monitoring service may add, modify, or remove a branch in an effort to improve the current neural network's accuracy. When the remote monitoring service determines that the new neural network is capable of more accurately classifying arrythmias, the remote monitoring service deploys a new branch to add to the current neural network of modular machine learning architecture 400. The new branch may be a replacement to a current branch. In some example, the remote monitoring service deploys an update comprising a new neural network to replace the current neural network. The remote monitoring service may package a new module to include the new neural network as unmasked in addition to a neural network for each other arrythmia type. It should be noted, the remote monitoring service may identify and then, deploy new neural networks to replace current ones in use at modular machine learning architecture 400 based on programming, monitoring reasons, future scientific discovery, and other reasons.

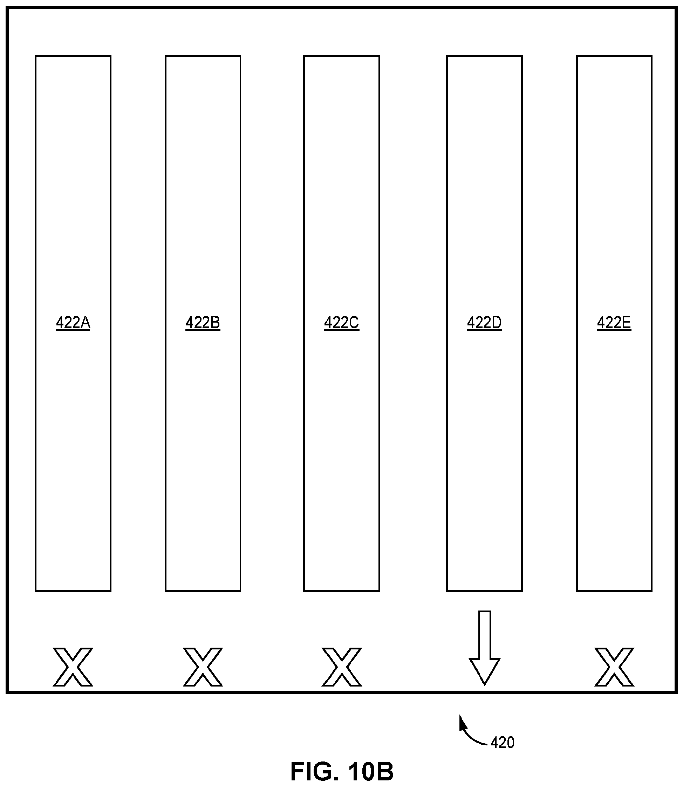

FIG. 10B is an illustration of an example module 420 of the modular machine learning architecture where each module includes a plurality of machine learning models (e.g., neural networks) even though only one model's prediction value is used. For example, module 420 includes machine learning models 422A-422E (collectively, "models 422"). As described herein, a component neural network's output class is a specific cardiac arrhythmia type and in example module 420, five different arrhythmia types are predicted by five different component neural networks 422, however, only one arrhythmia type's prediction is used in determining whether the patient has that arrhythmia type (e.g., and for generating output). Other modules in the same architecture are configured with multiple neural networks similar to module 420 but are dedicated to predicting a different arrhythmia type than module 420.

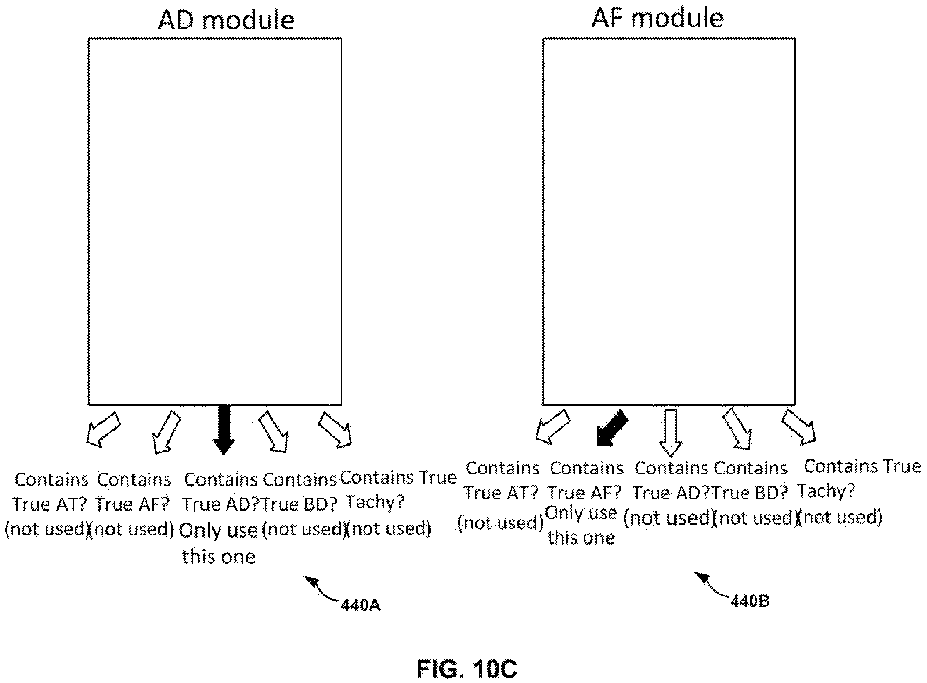

In some examples, module 420 discards all but one arrhythmia type's prediction value and produces a predictive value for one specific arrhythmia type. Hence, FIG. 10B does not illustrate an ensembling neural network for combining output prediction values because all but one network's output class is masked and/or discarded. It should be noted that example module 420 represents one example (multi-)neural network configuration and other modular machine learning architectures employ an example ensembling neural network to produce the predictive value for the one specific arrhythmia type, for example, by selecting a specific neural network's predictive value (with possible modification) or from combining values from two or more neural networks. The specific neural network may be an up-to-date component model dedicated to predicting the one specific arrhythmia type, and the ensembling network may be configured to evaluate that neural network's predictive value using one or more metrics, such as a metric indicating a likelihood of a false positive/false negative (i.e., sensitivity/specificity). In other examples, the specific neural network includes interconnected model components with another network and together, predict two or more arrhythmia types (e.g., as illustrated in FIGS. 10C-D) in which case the ensembling network may evaluate and/or select the prediction regarding the one specific arrhythmia type (discarding the other arrhythmia type's prediction) or (somehow) combine both predictions to determine the prediction regarding the one specific arrhythmia type.

As an alternative to independent neural networks 422 as illustrated in FIG. 10B, example module 420 may include a single ensemble of two or more (e.g., committee) networks and an ensembling (e.g., board) network where each committee network produces respective predictive values for two or more arrhythmia types and the board network evaluates each committee network's predictive values to generate a prediction for one specific arrhythmia type. The board network may also generate a prediction for one or more other arrhythmia type but those are discounted and not used as a positive/negative detection.

As one benefit to the medical devices, when an update having a new neural network is received from a remote monitoring service, the medical devices only have to update a single module with the new neural network. Because the other modules will not use their old neural networks for classification, the medical device do not have update those modules. With respect to example module 420, each masked neural network in the ensemble

FIG. 10C is an illustration of example modules 440A and 440B configured to detect AD events and AF events, respectively and when combined with BD, Tachy, and AT modules, form a portion of modular machine learning architecture 400. Example modules 440A and 440B are illustrated in FIG. 10C without the BD, Tachy, and AT modules for sake of brevity. In a manner similar to module 420 of FIG. 10B, both example modules 440A and 440B include machine learning models (e.g., neural networks) for predicting different arrhythmia types but each is configured to output one prediction (e.g., prediction value) for that module's respective arrhythmia type. In contrast to module 420, example modules 440A and 440B each include a neural network ensemble consisting of versions of each neural network but only a current version for the neural network corresponding to classifying cardiac EGM data as AD events and AF events, respectively. In example module 440A and module 440B, only the neural networks for classifying AD events and AF events, respectively, are unmasked and allowed to predict a likelihood for that event in a sample of cardiac EGM data. As depicted in FIG. 10C, other neural networks are not used.

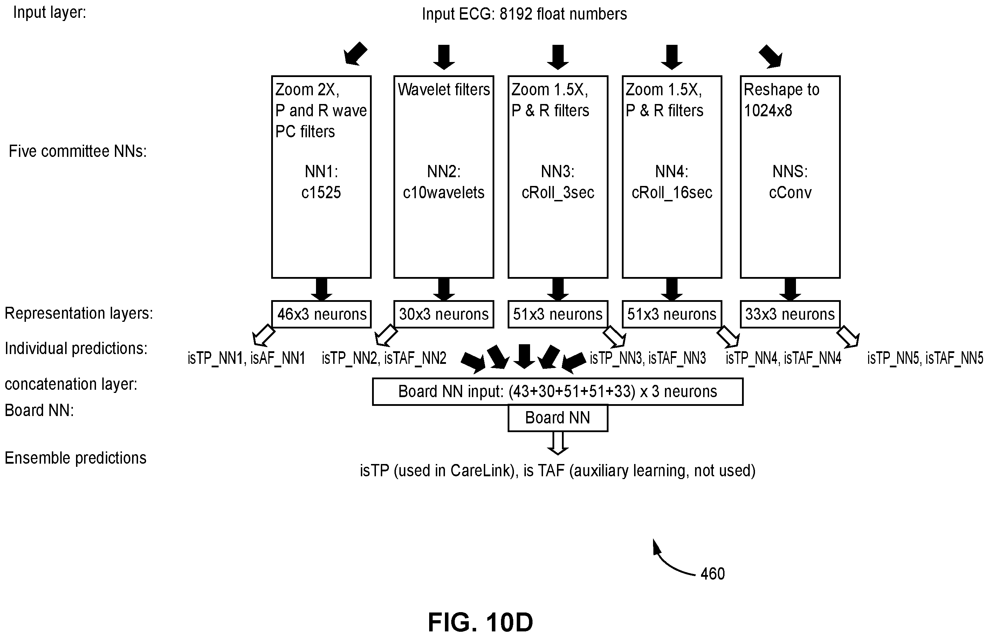

FIG. 10D is an illustration of an example module (or a portion thereof) undergoing an example detection analysis for a particular cardiac arrhythmia type while masking one or more other cardiac arrhythmia types. FIG. 10D illustrates the example detection analysis as detection analysis 460 for a (e.g., suspected) True Pause episode for which detection analysis 460 generates a probability represented by "isTP" as a prediction value (e.g., a likelihood) for the episode having True Pause. As described herein, detection analysis 460 invokes a board network that produces, as a part of an ensemble prediction, a probability for True Atrial Fibrillation (isTAF) but that predictive value is not used (e.g., output) for detecting a True Atrial Fibrillation episode.

When compared to example module 420 of FIG. 10B, the example module of FIG. 10 is a network ensemble composed of five committee neural networks, each producing a component (e.g., partial) predictive value for True Pause, and a board network configured to combine the five component predictive values into the above probability ("isTP") for output as the example module's prediction.

The present disclosure describes how an example detection analysis, such as detection analysis 460, may generate useful results for testing different versions of neural networks, such as those in the example module of FIG. 10D. In addition, some examples employ detection analysis 460 to evaluate specific model components (e.g., neural network layers) for their impact on the model's prediction, for example, in terms of a likelihood for increasing or decreasing true predictions or false determinations.

With respect to a neural network as the model being tested, detection analysis 460 may be used to evaluate, as one example neural network layer, a linear zoom layer. In general, a linear zoom layer is configured to find the max value of a waveform in episode data, clip the waveform at a designated value, and then, renormalize that value to 1. In an example of a 2 fold linear zoom layer, an input layer may include values [-1 1 2 6 0] with a max value of 6 and therefore, the input layer is clipped by an amount of 6/2=3 and then, divided by 3. The waveform includes plotted points (e.g., 2D points) and the linear zoom layer clips any plotted point above 3 such that the new max is 3 and an output layer includes a waveform represented by [-0.33 0.33 0.66 1 0]. As depicted in FIG. 10C, NN1 has zoom 2X. NN3 and 4 has zoom 1.5X. Other NN has no zooming layer. This layer is configured to enable the NN to differentiate P and dropped R waves better. This diversified the perceptions of each NN and resulting in a better ensemble.

As another example neural network layer to be tested, a time shifted residual layer may be configured as a modified regular residual layer that adds a shallower layer or branch as an output class (e.g., layer). For example, given resource availability and consumption information, the time shifted residual layer may, at a certain depth, connect (e.g., an output of ) a layer to (e.g., an input) of a (e.g., downstream) layer, such as when available resources are scarce. The second layer is shallower than the first layer and either in a same neural network (e.g., component model or committee network) or in another network (e.g., a board network). The time shifted residual layer may connect (e.g., the output of) the second layer back to (e.g., the input of) the first layer, facilitating circular time-shifted output for the first layer. By doing so, the time shifted residual layer may dynamically alter the neural network to avoid errors caused by resource deficiencies.

For example, in a module including five neural networks, NN1, NN2, NN#, NN4, and NN1, at least one network includes second layer output = [1 2 3 4] and first layer output = [5 6 7 8]. As a result, a regular residual layer = [1 2 3 4] + [5 6 7 8] = [6 8 10 12]. The example time shifted residual layer with 3 point circular shift at deeper layer would be [1 2 3 4] + [6 7 8 5] = [7 9 11 9]. NN1 and NN2 have regular residual layer. NN3 and NN4 have time shifted residual layer. NN5 has no residual layer. In details, NN3 and NN4 both have four time-shifted residual layers. NN3 uses time shifts of 3, 6, 9, and 12 seconds. NN4 uses time shifts of 16, 32, 48, and 0 second. This is designed in house to help NN3 and NN4 to compare the unstable/accelerating RR intervals after the true pause to the RR intervals before the true pause.

In order to mimic arrythmia detection of the mind of a human expert, the time shifted residual layer may add a layer (e.g., output layer) at a lower depth in the network and configure that layer to compute a prediction value for evaluation. This predication value may be considered a partial value or initial estimate after a preliminary evaluation (e.g., board decision). One example use of the above value is to test the neural network's performance (e.g., accuracy at the lower depth) given current/actual resource consumption. In some examples, it may be determined that at the certain depth of the above added layer, the network produces suitable and accurate prediction values; for at least this reason, the added layer may proceed (e.g., time shift) to the network's output layer/class and avoid unnecessary resource consumption. In this manner, the added layer omits a number of layers and saves considerable resources. The example time shift residual layer may add additional layers to the above layer, for instance, creating a chain of layers and further shifting the network.

A number of principal component and wavelet filtering layers may be tested using detection analysis 460. In one example, each layer includes one or more filters to differentiate R waves, PR intervals, and P waves, using principal components of LINQ waveforms and wavelets filters. This is an improvement over using randomly initialized filters in convolutional neural networks. The reasoning behind using pre-determined (as opposed of randomly initialized) filters is that, theoretically, using application-specific filters would increase the performance in that application but lose performance in other applications. The goal of these designed filters is to feed domain specific knowledge and decrease model parameter and thus decrease generalization errors. Principal component and wavelet filters outperform randomly initialized filters used in known architectures. In FIG. 10D, NN1, NN3, and NN4 utilize principal components filters. NN2 utilize wavelet filters. NN5 is the smallest of the five and utilize randomly initialized filters to provide varieties in representations. The predefined filters mimic the natural education curriculum of how human ECG adjudicator was trained, they are trained to read P and R waves before reading the rhythm. These differences in five NN diversified the perceptions of each NN and resulting in an improved network ensemble.

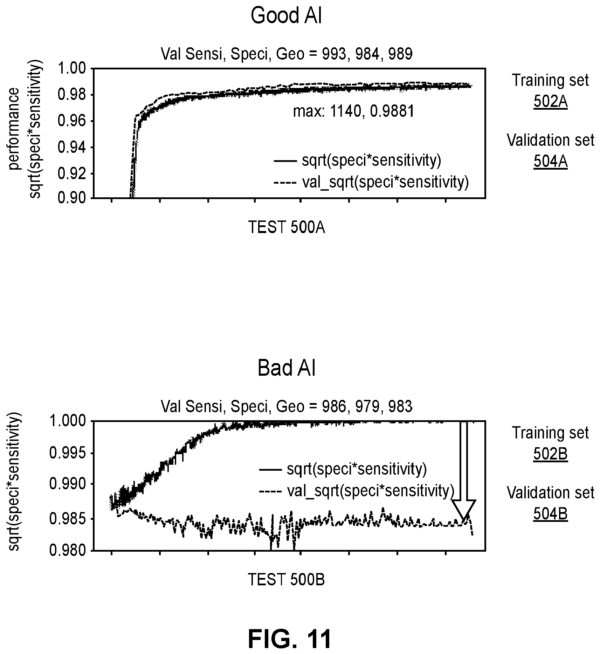

FIG. 11 includes illustrations of graphs depicting tests of machine learning models, in accordance with one or more examples of the present disclosure. Each illustration generally plots learning curves of geometric mean of sensitivity and specificity as a qualitative measurement of a neural network. Other metric values such accuracy may be employed to evaluate an aspect of the neural network's quality and then, compare that neural network with another model based on that quality.

In general, test 500A and test 500B refer to evaluations of respective neural networks. Training set 502A and training set 502B represent datasets of input data (e.g., cardiac EGM data) to the respective neural networks for the purpose of learning a classifier. Training may build a neural network such that, over time, the neural network predicts true positives at a higher rate. Hidden layers of the neural network include various criteria, weights, mathematical functions, probability distributions, and/or the like for computing a prediction value indicative of a likelihood that an input data sample is evidence of a true positive. Training may adapt the hidden layers to classifying the training dataset. When the neural network is sufficiently trained, validation may verify the neural network's true positive prediction rate. Similar to training sets 502, validation set 504A and validation set 504B represent datasets of input data to the respective neural networks and may help adapt each neural network to more accurately predict true positives. On the other hand, it is possible for a neural network to fail the validation, for example, if the hidden layers are inadequate.

As illustrated in FIG. 11, test 500A and test 500B demonstrate that the respective neural networks perform relatively at a same level with respect to training set 502A and training set 502B but deviate performance-wise with respect to validation set 504A and validation set 504B. Test 500A demonstrates that the neural network successfully completed training but failed validation, and test 500B demonstrates that the neural network successfully completed both training and validation. Both tests 500 include metric values (e.g., sensitivity and specificity) to evaluate the respective neural networks with respect to false determinations, such as false positives and false negatives. In addition, each of tests 500 indicates, as another metric value, a geometric mean that is computed from a square root of a product of sensitivity and specificity. The geometric mean of any neural network identifies a performance quality which the sensitivity and specificity would not suggest and for at least that reason, enables differentiation with other networks.

To further improve training and validation of neural networks, the present disclosure describes example where, for both test 500A and test 500B, processing circuitry 98 of server 94 may terminate the training of the respective neural network before completing training set 502A. As one example, processing circuitry 98 may stop (e.g., plateau-stop) the training when a performance metric reaches a plateau (e.g., a threshold value for the geometric mean) or a convergence point (e.g., when the training and validation learning curves converge). Test 500A depicts an example where monitoring service 6 can apply plateau-stopping and receive benefits. As one benefit, monitoring service 6 realizes savings from conserving computing resources that were allocated to the training.

There are additional reasons for halting the training of any neural network; as an example, processing circuitry 98 may early stop the training when a performance metric reaches a divergence point (e.g., when the training and validation learning curves diverge). Generalization (or out of sample) error-an amount at which the performance metric diverges (e.g., a difference between the training and validation learning curves)-represents a risk that the neural network is poorly trained and would perform poorly. Alternatively, processing circuitry 98 may halt training when an amount of divergence between the training and validation learning curves exceeds a threshold generalization error because the neural network cannot be reformed.

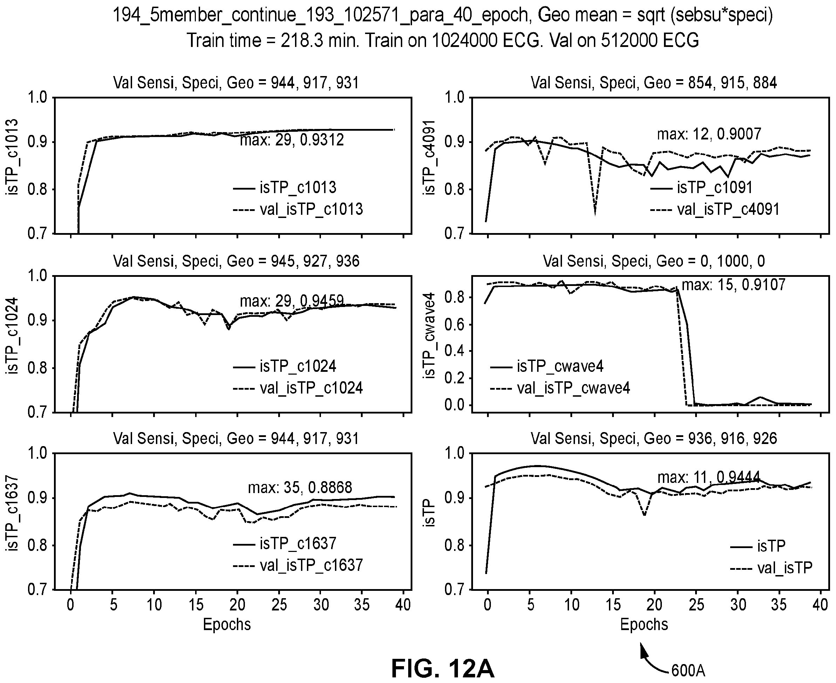

FIGS. 12A-B are both illustrations of tests 600A and 600B on an ensemble configured to classify a particular cardiac event. Although the following description refers to a neural networks ensemble, the ensemble of tests 600A and 600B may be an ensemble of any type of machine learning model.

As illustrated in FIG. 12A, the neural network ensemble is labelled "isTP" where TP refers to true positive and each component neural network is labeled "isTP" following by an identifier (e.g., "c1013"). The neural network ensemble "isTP" combines the component models using an ensembling model; for example, the ensembling model receives, as input, prediction values from the component neural networks and then, combines the prediction values using some technique (e.g., while reducing an error such as a generalization error). One example technique employs methods of model averaging.

As further illustrated in FIG. 12A, the neural network ensemble "isTP" has a geometric mean of 926 which is lower than component neural network "isTP_c1024" and therefore, does not improve the overall true positive prediction rate. In addition, component neural network "isTP_cwave4" has a geometric mean of 0 which is indicative of a poor performing component model. In this example, the geometric mean is a better performance metric than specificity which is 1000.

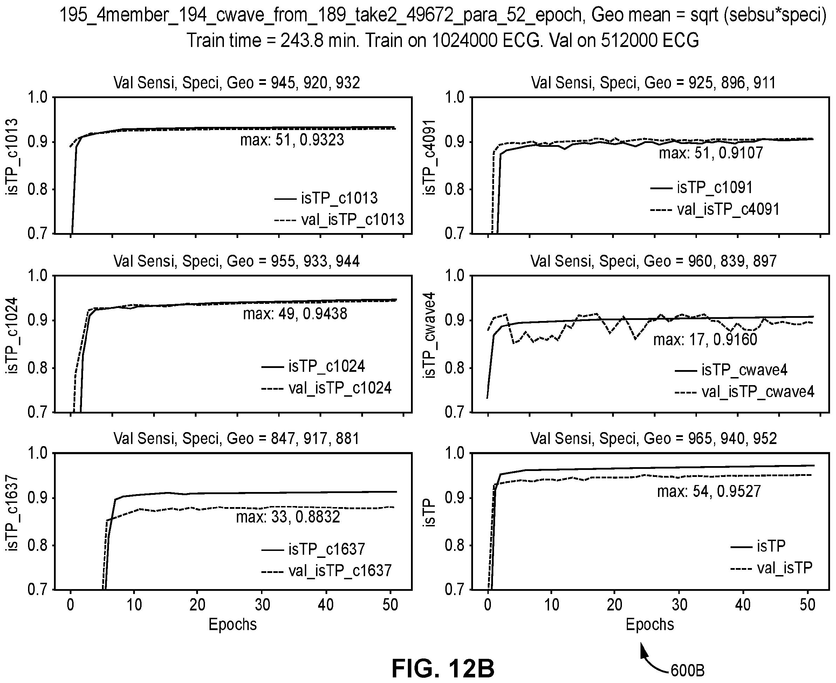

As illustrated in FIG. 12B, monitoring service 6 updates component neural network "isTP_cwave4" to improve upon that network's performance. For that model, performance metrics of sensitivity, specificity, and the geometric mean have improved to 960, 839, and 897, respectively. Furthermore, the overall performance of the neural network ensemble "isTP" has improved to the point where the ensemble is better than any individual component neural network and therefore, provides a performance gain over than component model. In some examples, by replacing or modifying the component neural network "isTP_cwave4", the overall true positive prediction rate of the entire neural network ensemble "isTP" is improved. For example, monitoring service 6 may upgrade component neural network "isTP_cwave4" to remove an unstable GRU layer and then, distribute that upgraded component neural network as an update to a modular machine learning architecture in use at IMD 10.

The order and flow of the operation illustrated in FIGS. 6 and 7 are examples. In other examples according to this disclosure, more or fewer thresholds may be considered. Further, in some examples, processing circuitry may perform or not perform the methods of FIG. 6 and FIG. 7, or any of the techniques described herein, as directed by a user, e.g., via external device 12, server 94, or computing devices 99. For example, a patient, clinician, or other user may turn on or off functionality for identifying changes in patient health (e.g., using Wi-Fi or cellular services) or locally (e.g., using an application provided on a patient's cellular phone or using a medical device programmer).

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as physician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, DRAM, SRAM, magnetic discs, optical discs, flash memories, or forms of EPROM or EEPROM. The instructions may be executed to support one or more aspects of the functionality described in this disclosure.

In addition, in some aspects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements. The techniques of this disclosure may be implemented in a wide variety of devices or apparatuses, including an IMD, an external programmer, a combination of an IMD and external programmer, an integrated circuit (IC) or a set of ICs, and/or discrete electrical circuitry, residing in an IMD and/or external programmer.

## Claims

1. A medical system comprising:
one or more sensors (62) configured to sense at least one physiological parameter corresponding to cardiac physiology of a patient (4);
sensing circuitry (52) configured to generate patient physiological data based on the sensed physiological parameter, the patient physiological data comprising cardiac EGM data for the patient; and
processing circuitry (50) configured to:
detect a cardiac event type for the patient based on a classification of the patient physiological data in accordance with a modular machine learning architecture (68), wherein the modular machine learning architecture comprises, for each of a plurality of cardiac event types, a respective component model for classifying the cardiac EGM data as evidence of that respective one of the plurality of cardiac event types; and
generate for display output data indicative of a positive detection of the cardiac event type.

2. The medical system of claim 1, comprising at least one of an implantable device (10), a wearable device, a pacemaker/defibrillator, or a ventricular assist device (VAD) that comprises the one or more sensors and the sensing circuitry.

3. The medical system of claim 1, wherein the one or more sensors comprise one or more electrodes (16) for sensing electrical activity of a heart of the patient, wherein the sensing circuitry is further configured to generate data to record digitized signals representing the electrical activity,
wherein the processing circuitry is further configured to identify one or more waveforms from the data recording the digitized signals, apply the modular machine learning architecture to the one or more waveforms, and based on prediction values of the modular machine learning architecture, determine whether the one or more waveforms indicate the cardiac event type.

4. The medical system of claim 1, wherein each of a plurality of modules (70) of the modular machine learning architecture classifies a respective cardiac arrhythmia type and, in that module, an ensemble comprises a component neural network for predicting a likelihood of each cardiac arrhythmia type.

5. The medical system of claim 4, wherein each component neural network is configured to classify the cardiac EGM data as one of: atrial fibrillation, atrial flutter, atrial flutter and atrial fibrillation, atrioventricular block, intraventricular conduction delay, premature contraction, premature ventricular contraction, premature atrial or atrioventricular junctional contraction, asystole/pause sinus bradycardia, sinus rhythm, sinus tachycardia, supraventricular tachycardia, and ventricular fibrillation.

6. The medical system of claim 4, wherein each component neural network comprises a neural network ensemble configured to determine respective values for a plurality of cardiac arrhythmia types, wherein the processing circuitry is further configured to generate, based on only a respective value for the corresponding cardiac arrhythmia type for that module, data indicative of a prediction of the corresponding cardiac arrhythmia type.

7. The medical system of claim 1, wherein to detect the cardiac event type, the processing circuitry is configured to verify, with a remote monitoring service, the modular machine learning architecture as having a current architecture and a current component model for classifying the cardiac EGM data as evidence of each of the respective cardiac event types.

8. The medical system of claim 1, wherein to generate, for display, output data, the processing circuitry is configured to generate output data indicative of a waveform of the cardiac EGM data spanning an occurrence of the cardiac event type.

9. The medical system of claim 1, wherein the processing circuitry is further configured to receive, from a remote monitoring service, an update to the modular machine learning architecture, wherein the update comprises a new branch of a new component model, the new component model, or a new module with the new component model.

10. The medical system of claim 1, wherein to detect the cardiac event type, the processing circuitry is configured to update the modular machine learning architecture by one or more of: removing at least a portion of a component model, modifying the at least a portion of a component model, replacing the at least a portion of a component model, adding a new component model to the component models of an ensemble, or adding a new branch to one of the component models.

11. The medical system of claim 1, wherein to detect the cardiac event type, the processing circuitry is configured to combine predictions from the respective component models that form the modular machine learning architecture to determine whether to classify the cardiac EGM data as a cardiac arrhythmia.

12. A medical system configured to run a remote monitoring service (6) for a plurality of medical devices, comprising:
communication circuitry (54) configured to exchange data with the medical devices corresponding to the remote monitoring service, wherein a portion of the data corresponds to a modular machine learning architecture (68) that the medical devices employs to classify cardiac EGM data as evidence of an episode corresponding to one or more of a plurality of cardiac arrhythmia types; and
processing circuitry (80) operative to execute logic for the remote monitoring service, wherein the logic is configured to:
maintain, in a storage device (96), a most recent version of the modular machine learning architecture, wherein the modular machine learning architecture comprises, in each of a plurality of modules, a respective neural network for predicting a likelihood that any given cardiac EGM sample indicates a respective cardiac arrhythmia type; and
in response to an update to the modular machine learning architecture, deploy, to the medical devices via the communication circuitry, a new module that corresponds to the update.

13. The medical system of claim 12, wherein the update comprises a new neural network that is configured to predict a likelihood that any given cardiac EGM sample indicates a particular cardiac arrhythmia type and the processing circuitry is further configured to communicate, to the medical devices via the communication circuitry, the new neural network.

14. The medical system of claim 13, wherein the processing circuitry is further configured to train the new neural network using patient cardiac activity data.

15. The medical system of claim 13, wherein the processing circuitry is further configured to validate the new neural network based on a geometric mean that is computed from a specificity metric value and a sensitivity metric value.

## Patentansprüche

1. Medizinisches System, umfassend:
einen oder mehrere Sensoren (62), die dazu konfiguriert sind, mindestens einen physiologischen Parameter zu erfassen, der der Herzphysiologie eines Patienten (4) entspricht;
eine Sensorschaltungsanordnung (52), die dazu konfiguriert ist, physiologische Patientendaten basierend auf den erfassten physiologischen Parametern zu generieren, wobei die physiologischen Patientendaten EGM-Herzdaten für den Patienten umfassen; und
eine Verarbeitungsschaltungsanordnung (50), die zu Folgendem konfiguriert ist:
Erkennen eines Herzereignistyps für den Patienten basierend auf einer Klassifizierung der physiologischen Patientendaten gemäß einer modularen Architektur für maschinelles Lernen (68), wobei die modulare Architektur für maschinelles Lernen für jeden einer Mehrzahl von Herzereignistypen ein jeweiliges Komponentenmodell zum Klassifizieren der EGM-Herzdaten als Nachweis für diesen jeweiligen der Mehrzahl von Herzereignistypen umfasst; und
Generieren von Ausgabedaten zur Anzeige, die eine positive Erkennung des Herzereignistyps anzeigen.

2. Medizinisches System nach Anspruch 1, umfassend mindestens eine von einer implantierbaren Vorrichtung (10), einer am Körper tragbaren Vorrichtung, einem Schrittmacher/Defibrillator oder einer ventrikulären Unterstützungsvorrichtung (VAD), die den einen oder die mehreren Sensoren und die Sensorschaltungsanordnung umfasst.

3. Medizinisches System nach Anspruch 1, wobei der eine oder die mehreren Sensoren eine oder mehrere Elektroden (16) zum Erfassen der elektrischen Aktivität des Herzens des Patienten umfassen, wobei die Sensorschaltungsanordnung ferner dazu konfiguriert ist, Daten zu generieren, um digitalisierte Signale aufzuzeichnen, die die elektrische Aktivität darstellen, wobei die Verarbeitungsschaltungsanordnung ferner dazu konfiguriert ist, eine oder mehrere Wellenformen aus den Daten zu identifizieren, die die digitalisierten Signale aufzeichnen, die modulare Architektur für maschinelles Lernen auf die eine oder die mehreren Wellenformen anzuwenden und basierend auf Vorhersagewerten der modularen Architektur für maschinelles Lernen zu bestimmen, ob die eine oder die mehreren Wellenformen den Herzereignistyp anzeigen.

4. Medizinisches System nach Anspruch 1, wobei jedes einer Mehrzahl von Modulen (70) der modularen Architektur für maschinelles Lernen einen jeweiligen Herzrhythmusstörungstyp klassifiziert und in diesem Modul ein Ensemble ein neuronales Komponentennetzwerk zum Vorhersagen einer Wahrscheinlichkeit jedes Herzrhythmusstörungstyps umfasst.

5. Medizinisches System nach Anspruch 4, wobei jedes neuronale Komponentennetzwerk dazu konfiguriert ist, die EGM-Herzdaten als eines von Folgenden zu klassifizieren:
Vorhofflimmern, Vorhofflattern, Vorhofflattern und Vorhofflimmern, atrioventrikulären Block, intraventrikuläre Leitungsverzögerung, vorzeitige Kontraktion, vorzeitige Ventrikelkontraktion, vorzeitige junktionale Vorhof- oder Vorhofkammerkontraktion, Asystolie/Pause, Sinusbradykardie, Sinusrhythmus, Sinustachykardie, supraventrikuläre Tachykardie, und Kammerflimmern.

6. Medizinisches System nach Anspruch 4, wobei jedes neuronale Komponentennetzwerk ein neuronales Netzwerk-Ensemble umfasst, das dazu konfiguriert ist, jeweilige Werte für eine Mehrzahl von Herzrhythmusstörungstypen zu bestimmen, wobei die Verarbeitungsschaltungsanordnung ferner dazu konfiguriert ist, basierend auf nur einem jeweiligen Wert für den entsprechenden Herzrhythmusstörungstyp für dieses Modul Daten zu generieren, die eine Vorhersage des entsprechenden Herzrhythmusstörungstyps anzeigen.

7. Medizinisches System nach Anspruch 1, wobei zum Erkennen des Herzereignistyps die Verarbeitungsschaltungsanordnung dazu konfiguriert ist, mit einem Fernüberwachungsdienst zu überprüfen, ob die modulare Architektur für maschinelles Lernen eine aktuelle Architektur und ein aktuelles Komponentenmodell zum Klassifizieren der EGM-Herzdaten als Nachweis für jeden der jeweiligen Herzereignistypen aufweist.

8. Medizinisches System nach Anspruch 1, wobei zum Generieren von Ausgabedaten zur Anzeige die Verarbeitungsschaltungsanordnung dazu konfiguriert ist, Ausgabedaten zu generieren, die eine Wellenform der EGM-Herzdaten anzeigen, die ein Auftreten des Herzereignistyps umfassen.

9. Medizinisches System nach Anspruch 1, wobei die Verarbeitungsschaltungsanordnung ferner dazu konfiguriert ist, von einem Fernüberwachungsdienst eine Aktualisierung der modularen Architektur für maschinelles Lernen zu empfangen, wobei die Aktualisierung einen neuen Zweig eines neuen Komponentenmodells, das neue Komponentenmodell oder ein neues Modul mit dem neuen Komponentenmodell umfasst.

10. Medizinisches System nach Anspruch 1, wobei zum Erkennen des Herzereignistyps die Verarbeitungsschaltungsanordnung dazu konfiguriert ist, die modulare Architektur für maschinelles Lernen durch eines oder mehreres von Folgendem zu aktualisieren: Entfernen mindestens eines Teils eines Komponentenmodells, Modifizieren des mindestens einen Teils eines Komponentenmodells, Ersetzen des mindestens einen Teils eines Komponentenmodells, Hinzufügen eines neuen Komponentenmodells zu den Komponentenmodellen eines Ensembles oder Hinzufügen eines neuen Zweiges zu einem der Komponentenmodelle.

11. Medizinisches System nach Anspruch 1, wobei zum Erkennen des Herzereignistyps die Verarbeitungsschaltungsanordnung dazu konfiguriert ist, Vorhersagen aus den jeweiligen Komponentenmodellen, die die modulare Architektur für maschinelles Lernen bilden, zu kombinieren, um zu bestimmen, ob die EGM-Herzdaten als Herzrhythmusstörung klassifiziert werden sollen.

12. Medizinisches System, das dazu konfiguriert ist, einen Fernüberwachungsdienst (6) für eine Mehrzahl von medizinischen Vorrichtungen auszuführen, und Folgendes umfasst:
eine Kommunikationsschaltungsanordnung (54), die dazu konfiguriert ist, Daten mit den medizinischen Vorrichtungen auszutauschen, die dem Fernüberwachungsdienst entsprechen, wobei ein Teil der Daten einer modularen Architektur für maschinelles Lernen (68) entspricht, die die medizinischen Vorrichtungen zum Klassifizieren von EGM-Herzdaten als Nachweis einer Episode einsetzen, die einem oder mehreren einer Mehrzahl von Herzrhythmusstörungstypen entspricht; und
eine Verarbeitungsschaltungsanordnung (80), die dazu ausgelegt ist, Logik für den Fernüberwachungsdienst auszuführen, wobei die Logik zu Folgendem konfiguriert ist:
Unterhalten einer neuesten Version der modularen Architektur für maschinelles Lernen in einer Speichervorrichtung (96), wobei die modulare Architektur für maschinelles Lernen in jedem einer Mehrzahl von Modulen ein jeweiliges neuronales Netzwerk zum Vorhersagen der Wahrscheinlichkeit umfasst, dass eine gegebene EGM-Herzprobe einen jeweiligen Herzrhythmusstörungstyp anzeigt; und
in Reaktion auf eine Aktualisierung der modularen Architektur für maschinelles Lernen Bereitstellen eines neuen Moduls, das der Aktualisierung entspricht, für die medizinischen Vorrichtungen über die Kommunikationsschaltungsanordnung.

13. Medizinisches System nach Anspruch 12, wobei die Aktualisierung ein neues neuronales Netzwerk umfasst, das dazu konfiguriert ist, eine Wahrscheinlichkeit vorherzusagen, dass eine gegebene EGM-Herzprobe einen bestimmten Herzrhythmusstörungstyp anzeigt, und die Verarbeitungsschaltungsanordnung ferner dazu konfiguriert ist, das neue neuronale Netzwerk über die Kommunikationsschaltung an die medizinischen Vorrichtungen zu kommunizieren.

14. Medizinisches System nach Anspruch 13, wobei die Verarbeitungsschaltungsanordnung ferner dazu konfiguriert ist, das neue neuronale Netzwerk unter Verwendung von Herzaktivitätsdaten des Patienten zu trainieren.

15. Medizinisches System nach Anspruch 13, wobei die Verarbeitungsschaltungsanordnung ferner dazu konfiguriert ist, das neue neuronale Netzwerk basierend auf einem geometrischen Mittelwert zu validieren, das aus einem Spezifitätsmetrikwert und einem Sensitivitätsmetrikwert berechnet wird.

## Revendications

1. Système médical comprenant :
un ou plusieurs capteurs (62) configurés pour détecter au moins un paramètre physiologique correspondant à la physiologie cardiaque d'un patient (4) ;
un circuit de détection (52) configuré pour générer des données physiologiques de patient sur la base du paramètre physiologique détecté, les données physiologiques de patient comprenant des données EGM cardiaques pour le patient ; et
un circuit de traitement (50) configuré pour :
détecter un type d'événement cardiaque pour le patient sur la base d'une classification des données physiologiques de patient conformément à une architecture modulaire d'apprentissage automatique (68), l'architecture modulaire d'apprentissage automatique comprenant, pour chacun d'une pluralité de types d'événement cardiaque, un modèle de composant respectif pour classifier les données EGM cardiaques comme preuve de ce type respectif parmi la pluralité de types d'événement cardiaque ; et
générer des données de sortie d'affichage indiquant une détection positive du type d'événement cardiaque.

2. Système médical selon la revendication 1, comprenant au moins l'un d'un dispositif implantable (10), d'un dispositif portable, d'un stimulateur/défibrillateur cardiaque ou d'un dispositif d'assistance ventriculaire (VAD) qui comprend le ou les capteurs et le circuit de détection.

3. Système médical selon la revendication 1, le ou les capteurs comprenant une ou plusieurs électrodes (16) pour détecter l'activité électrique d'un cœur du patient, le circuit de détection étant en outre configuré pour générer des données pour enregistrer des signaux numérisés représentant l'activité électrique,
le circuit de traitement étant en outre configuré pour identifier une ou plusieurs formes d'onde à partir des données enregistrant les signaux numérisés, appliquer l'architecture modulaire d'apprentissage automatique à la ou aux formes d'onde, et sur la base de valeurs de prédiction de l'architecture modulaire d'apprentissage automatique, déterminer si la ou les formes d'onde indiquent le type d'événement cardiaque.

4. Système médical selon la revendication 1, chacun d'une pluralité de modules (70) de l'architecture modulaire d'apprentissage automatique classifiant un type d'arythmie cardiaque respectif et, dans ce module, un ensemble comprenant un réseau neuronal de composant pour prédire une probabilité de chaque type d'arythmie cardiaque.

5. Système médical selon la revendication 4, chaque réseau neuronal de composant étant configuré pour classifier les données EGM cardiaques comme l'un de : fibrillation auriculaire, flutter auriculaire, flutter auriculaire et fibrillation auriculaire, bloc auriculo-ventriculaire, retard de conduction intraventriculaire, contraction prématurée, contraction ventriculaire prématurée, contraction prématurée de la jonction auriculaire ou auriculo-ventriculaire, asystolie/pause bradycardie sinusale, rythme sinusal, tachycardie sinusale, tachycardie supraventriculaire, et fibrillation ventriculaire.

6. Système médical selon la revendication 4, chaque réseau neuronal de composant comprenant un ensemble de réseau neuronal configuré pour déterminer des valeurs respectives pour une pluralité de types d'arythmie cardiaque, le circuit de traitement étant en outre configuré pour générer, sur la base seulement d'une valeur respective pour le type d'arythmie cardiaque correspondant pour ce module, des données indicatives d'une prédiction du type d'arythmie cardiaque correspondant.

7. Système médical selon la revendication 1, pour détecter le type d'événement cardiaque, le circuit de traitement étant configuré pour vérifier, avec un service de surveillance à distance, l'architecture modulaire d'apprentissage automatique comme ayant une architecture courante et un modèle de composant courant pour classifier les données EGM cardiaques comme preuve de chacun des types d'événement cardiaque respectifs.

8. Système médical selon la revendication 1, pour générer, pour affichage, des données de sortie, le circuit de traitement étant configuré pour générer des données de sortie indicatives d'une forme d'onde des données EGM cardiaques couvrant une occurrence du type d'événement cardiaque.

9. Système médical selon la revendication 1, le circuit de traitement étant en outre configuré pour recevoir, d'un service de surveillance à distance, une mise à jour de l'architecture modulaire d'apprentissage automatique, la mise à jour comprenant une nouvelle branche d'un nouveau modèle de composant, le nouveau modèle de composant, ou un nouveau module avec le nouveau modèle de composant.

10. Système médical selon la revendication 1, pour détecter le type d'événement cardiaque, le circuit de traitement étant configuré pour mettre à jour l'architecture modulaire d'apprentissage automatique par un ou plusieurs des éléments suivants : l'élimination d'au moins une partie d'un modèle de composant, la modification de l'au moins une partie d'un modèle de composant, le remplacement de l'au moins une partie d'un modèle de composant, l'ajout d'un nouveau modèle de composant aux modèles de composant d'un ensemble, ou l'ajout d'une nouvelle branche à l'un des modèles de composant.

11. Système médical selon la revendication 1, pour détecter le type d'événement cardiaque, le circuit de traitement étant configuré pour combiner des prédictions à partir des modèles de composant respectifs qui forment l'architecture modulaire d'apprentissage automatique pour déterminer s'il faut classifier les données EGM cardiaques comme une arythmie cardiaque.

12. Système médical configuré pour exécuter un service de surveillance à distance (6) pour une pluralité de dispositifs médicaux, comprenant :
des circuits de communication (54) configurés pour échanger des données avec les dispositifs médicaux correspondant au service de surveillance à distance, une partie des données correspondant à une architecture modulaire d'apprentissage automatique (68) que les dispositifs médicaux utilisent pour classer les données EGM cardiaques comme preuve d'un épisode correspondant à un ou plusieurs d'une pluralité de types d'arythmie cardiaque ; et
des circuits de traitement (80) fonctionnant pour exécuter une logique pour le service de surveillance à distance, la logique étant configurée pour :
maintenir, dans un dispositif de stockage (96), une version la plus récente de l'architecture modulaire d'apprentissage automatique, l'architecture modulaire d'apprentissage automatique comprenant, dans chacun d'une pluralité de modules, un réseau neuronal respectif pour prédire une probabilité qu'un échantillon EGM cardiaque donné indique un type d'arythmie cardiaque respectif ; et
en réponse à une mise à jour de l'architecture modulaire d'apprentissage automatique, déployer, sur les dispositifs médicaux par l'intermédiaire des circuits de communication, un nouveau module correspondant à la mise à jour.

13. Système médical selon la revendication 12, la mise à jour comprenant un nouveau réseau neuronal qui est configuré pour prédire une probabilité qu'un échantillon EGM cardiaque donné quelconque indique un type d'arythmie cardiaque particulier et le circuit de traitement étant en outre configuré pour communiquer, aux dispositifs médicaux par l'intermédiaire du circuit de communication, le nouveau réseau neuronal.

14. Système médical selon la revendication 13, le circuit de traitement étant en outre configuré pour entraîner le nouveau réseau neuronal en utilisant des données d'activité cardiaque du patient.

15. Système médical selon la revendication 13, le circuit de traitement étant en outre configuré pour valider le nouveau réseau neuronal sur la base d'une moyenne géométrique qui est calculée à partir d'une valeur métrique de spécificité et d'une valeur métrique de sensibilité.
